# FASCICULE DE BREVET EUROPEEN

(11) **EP 1 464 326 B1**
(45) Date de publication et mention de la délivrance du brevet: **25.10.2017**
(21) Numéro de dépôt: 04290869.9
(22) Date de dépôt: 01.04.2004
(51) Int. Cl.: A61K 8/41, A61K 8/49, A61K 8/73, A61K 8/81, A61K 8/87, A61Q 1/02, A61Q 5/06, A61Q 5/10

(54) **Composition de coloration des cheveux comprenant un colorant fluorescent et un polymère associatif**
Einen fluoreszierenden Farbstoff und ein assoziierendes Polymer enthaltende Haarfärbemischung
Hair colouring composition containing a fluorescent dye and an associative polymer

(30) Priorité: 01.04.2003 FR 0304035
(43) Date de publication de la demande: 06.10.2004
(73) Titulaire: L'Oréal, 75008 Paris (FR)
(72) Inventeur: Gourlaouen, Luc, 92600 Asnieres (FR); Samain, Henri, 91570 Bievres (FR)
(74) Mandataire: Wattremez, Catherine

(56) Documents cités:
- EP-A- 0 370 470
- EP-A- 0 970 684
- EP-A- 1 023 891
- EP-A- 1 099 437
- EP-A- 1 133 977
- EP-A- 1 191 041
- EP-A1- 1 179 336
- WO-A-01/43714
- WO-A-02/38115
- WO-A-99/13841
- WO-A-99/13845
- FR-A- 2 820 032
- FR-A1- 2 803 195
- DATABASE HCAPLUS [Online] ACS; 2000, XP002269220 extrait de STN Database accession no. 132:323123/DN & JP 2000 136340 A (PENTEL CO.) 16 mai 2000 (2000-05-16)
- "Fluorescent Whitening Agents" In: Kirk-Othmer: "Encyclopedia of Chemical technology, 4th edition", March 2005 (2005-03), Wiley Interscience Publication, New York vol. 11, pages 272-241,
- QIANGQIANG ZHAO ET AL: "Coloring properties of novel 1,4-distyrylbenzene and 4,4'-distyrylbiphenyl fluorescent brighteners and their arrangement in cotton and polyester fiber", CELLULOSE, vol. 21, no. 4, 6 May 2014 (2014-05-06), pages 2937-2950, XP055198137, ISSN: 0969-0239, DOI: 10.1007/s10570-014-0260-0

## Description

L'invention concerne une composition comprenant au moins un colorant fluorescent et au moins un polymère associatif particuliers ainsi que les procédés et dispositif mettant en oeuvre ces compositions. Elle concerne également l'utilisation de compositions comprenant au moins un colorant fluorescent et au moins un polymère associatif particuliers, pour colorer avec un effet éclaircissant les matières kératiniques humaines et plus particulièrement les fibres kératiniques comme les cheveux pigmentés ou colorés artificiellement, ainsi que la peau foncée.

Il est fréquent que les personnes ayant une peau foncée désirent s'éclaircir la peau et utilisent dans ce but des compositions cosmétiques ou dermatologiques contenant des agents de blanchiment.

Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.

Toutefois ces agents ne sont pas dépourvus d'inconvénients. En particulier, il est nécessaire de les utiliser de façon prolongée et en des quantités élevées, pour obtenir un effet de blanchiment de la peau. On n'observe pas un effet immédiat à l'application de compositions les comprenant.

En outre, l'hydroquinone et ses dérivés sont utilisés en quantité efficace pour voir apparaître un effet blanchissant. En particulier, l'hydroquinone est connue pour sa cytotoxicité vis-à-vis du mélanocyte.

Par ailleurs, l'acide kojique et ses dérivés présentent l'inconvénient d'être coûteux et de ne pouvoir, pour cette raison, être utilisés en quantité importante dans des produits à large diffusion commerciale.

Il subsiste donc le besoin de compositions cosmétiques permettant d'obtenir un teint plus clair, uniforme, homogène, d'aspect naturel, ces compositions présentant une transparence satisfaisante après application sur la peau.

Dans le domaine capillaire, Il existe principalement deux grands types de coloration capillaire.

Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.

Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.

Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis à vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La présente invention a pour objet de résoudre les problèmes mentionnés ci-dessus et notamment de proposer une composition qui présente une bonne affinité tinctoriale pour les matières kératiniques et notamment les fibres kératiniques, de bonne propriété de ténacités vis à vis des agents extérieurs, et en particulier vis-à-vis des shampooings, et qui permettent également d'obtenir un éclaircissement sans altération de la matière traitée, plus particulièrement la fibre kératinique.

Il a donc été trouvé de façon inattendue et surprenante que l'utilisation de colorants fluorescents, en particulier ceux dans la gamme des orangés, en présence de polymères associatifs particuliers, permettait d'atteindre ces objectifs.

La présente invention a donc pour premier objet une composition, comprenant, dans un milieu cosmétiquement acceptable, au moins un colorant fluorescent soluble dans ledit milieu et au moins un polymère associatif choisi parmi les dérivés de polyuréthanes associatifs, les dérivés de celluloses associatifs, les dérivés de polyvinyllactames associatifs et les dérivés de polyacides insaturés associatifs ; la composition ne comprenant pas, à titre d'agent fluorescent, du 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure ; lesdits colorants fluorescents sont tels que définis dans la revendication 1.

Un second objet de l'invention concerne un procédé pour colorer avec un effet éclaircissant les fibres kératiniques humaines, dans lequel on met en oeuvre les étapes suivantes :
a) on applique sur lesdites fibres une composition selon l'invention, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

Un autre objet de l'invention concerne l'utilisation d'une composition pour colorer avec un effet éclaircissant les matières kératiniques humaines, comprenant dans un milieu cosmétiquement acceptable, au moins un colorant fluorescent soluble dans ledit milieu et au moins un polymère associatif choisi parmi les dérivés de polyuréthanes associatifs, les dérivés de celluloses associatifs, les dérivés de polyvinyllactames associatifs et les dérivés de polyacides insaturés associatifs ledit colorant fluorescent étant tel que défini dans la revendication 34.

Les compositions de l'invention permettent en particulier une meilleure fixation du colorant fluorescent dans les matières kératiniques ce qui se traduit par un effet de fluorescence accru et à un effet d'éclaircissement supérieur à celui obtenu avec le colorant fluorescent utilisé seul.

On constate également une meilleure ténacité du résultat vis-à-vis des lavages ou shampooings.

Mais d'autres caractéristiques et avantages de la présente invention apparaîtront plus clairement à la lecture de la description et des exemples qui vont suivre.

A moins d'une indication différente, les bornes des gammes de valeurs qui sont données dans la description, sont incluses dans ces gammes.

Comme cela a été indiqué auparavant, la composition selon l'invention comprend au moins un colorant fluorescent particulier et au moins un polymère associatif particulier choisi parmi les dérivés de polyuréthanes associatifs, les dérivés de celluloses associatifs, les dérivés de polyvinyllactames associatifs et les dérivés de polyacides insaturés associatifs..

Il est précisé que les polymères associatifs sont des polymères hydrophiles capables, dans un milieu aqueux, de s'associer réversiblement entre eux ou avec d'autres molécules.

Leur structure chimique comprend plus particulièrement au moins une zone hydrophile et au moins une zone hydrophobe.

Par groupement hydrophobe, on entend un radical ou polymère à chaîne hydrocarbonée, saturée ou non, linéaire ou ramifiée.

Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Les polymères associatifs présents dans la composition selon l'invention peuvent être de nature non ionique, anionique, cationique ou amphotère.

Parmi les dérivés de polyuréthanes associatifs, on peut citer les copolymères anioniques obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique.

De tels sont notamment décrits dans EP 173109 et plus particulièrement dans l'exemple 3. Plus précisément, ce polymère est un terpolymère acide méthacrylique / acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi citer les polyuréthanes associatifs cationiques dont la famille a été décrite dans la demande FR 0009609. Elle peut être représentée plus particulièrement par la formule générale (Ia) suivante :

R-X-(P)ₙ-[L-(Y)ₘ]ᵣ-L'-(P')ₚ-X'-R' (Ia)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25 ;
n, m, et p valent chacun indépendamment des autres entre 0 et 1000 ;
la molécule contenant au moins une fonction amine protonée ou quaternisée et au moins un groupement hydrophobe.

Dans un mode de réalisation très avantageux, les seuls groupements hydrophobes de ces polyuréthanes sont les groupes R et R' situés aux extrémités de chaîne.

Selon un premier mode de réalisation préféré de l'invention, le polyuréthane associatif cationique correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée que dans la formule (Ia).

Selon un autre mode de réalisation préféré de l'invention, le polyuréthane associatif cationique correspond à la formule (Ia) ci-dessus dans laquelle :
R et R' représentent tous les deux indépendamment un groupement hydrophobe,
X, X' représentent chacun un groupe L", n et p valent 0, et L, L', L", Y et m ont la signification que dans la formule (Ia) indiquée.

Le fait que n et p valent 0 signifie que ces polymères ne comportent pas de motifs dérivés d'un monomère à fonction amine, incorporé dans le polymères lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Conformément à un autre mode de réalisation préféré de l'invention, le polyuréthane associatif cationique correspond à la formule (Ia) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée dans la formule (Ia).

La masse moléculaire moyenne en nombre des polyuréthanes associatifs cationiques est habituellement comprise entre 400 et 500000, en particulier entre 1000 et 400000 et idéalement entre 1000 et 300000 g/mol.

Lorsque X et/ou X' désignent un groupement comportant une amine tertiaire ou quaternaire, X et/ou X' peuvent représenter l'une des formules suivantes : 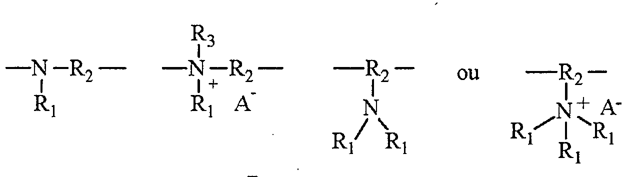 pour X 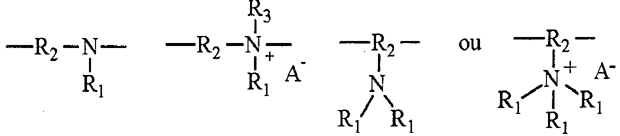 pour X' dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L'et L" représentent un groupe de formule : 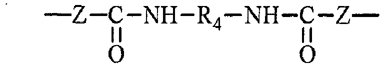 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 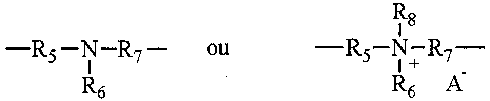 ou 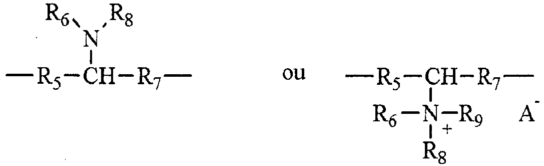 ou 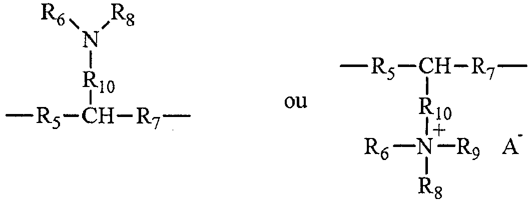 dans lesquelles :
R₅ et R₇ ont les mêmes significations que R₂ défini précédemment ;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P ;
et A⁻ est un contre-ion cosmétiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (Ia) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH, ou HZ-(P')ₚ-ZH

dans lesquelles Z, P, P', n et p sont tels que définis plus haut.

A titre d'exempleS de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (la) est un diisocyanate correspondant à la formule O=C=N-R₄-N=C=O dans laquelle R₄ est défini plus haut.

On peut citer notamment le méthylènediphényl-diisocyanate, le méthylène cyclohexane diisocyanate, l'isophorone-diisocyanate, le toluènediisocyanate, le naphtalène diisocyanate, le butanediisocyanate, l'hexanediisocyanate.

Un troisième composé entrant dans la préparation du polyuréthane de formule (Ia) est un composé hydrophobe destiné à former les groupes hydrophobes terminaux du polymère de formule (Ia).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné -hydroxyle. Le groupe hydrophobe du polyuréthane de formule (Ia) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemples, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères.

A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse. Bien que la présence d'un groupe Y hydrophile soit facultative, on préfère cependant des polyuréthanes associatifs cationiques comportant un tel groupe.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.

Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention peuvent aussi être choisis parmi ceux décrits dans l'article de G. Fonnum, J. Bakke et Fk. Hansen - Colloid Polym. Sci 271,380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Parmi les polymères dérivés de celluloses associatifs utilisables selon l'invention on peut citer :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaîne hydrophobe en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs utilisables dans le cadre de la présente invention, ces derniers peuvent notamment être choisis parmi ceux décrits dans FR 0101106.

Lesdits polymères sont plus particulièrement des polymères cationiques et comprennent :
- a) au moins un monomère de type vinyl lactame ou alkylvinyllactame;
- b) au moins un monomère de structures (II) ou (III) suivantes : 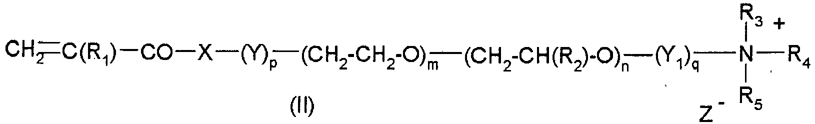 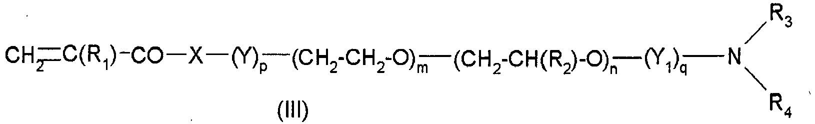 dans lesquelles :
   X désigne un atome d'oxygène ou un radical NR₆,
   R₁ et R₆ désignent, indépendamment l'un de l'autre, un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (IV) :

      -(Y₂)ᵣ-(CH₂-CH(R₇)-O)ₓ-R₈ (IV)
   Y, Y₁ et Y₂ désignent, indépendamment l'un de l'autre, un radical alkylène linéaire ou ramifié en C₂-C₁₆,
   R₇ désigne un atome d'hydrogène, ou un radical alkyle linéaire ou ramifié en C₁-C₄ ou un radical hydroxyalkyle linéaire ou ramifié en C₁-C₄,
   R₈ désigne un atome d'hydrogène ou un radical alkyle linéaire ou ramifié en C₁-C₃₀,
   p, q et r désignent, indépendamment l'un de l'autre, soit la valeur zéro, soit la valeur 1,
   m et n désignent, indépendamment l'un de l'autre, un nombre entier allant de 0 à 100,
   x désigne un nombre entier allant de 1 à 100,
   Z désigne un anion d'acide organique ou minéral,
   sous réserve que :
      - l'un au moins des substituants R₃, R₄, R₅ ou R₈ désigne un radical alkyle linéaire ou ramifié en C₉-C₃₀,
      - si m ou n est différent de zéro, alors q est égal à 1,
      - si m ou n sont égaux à zéro, alors p ou q est égal à 0.

Les polymères poly(vinyllactame) selon l'invention peuvent être réticulés ou non réticulés et peuvent aussi être des polymères blocs.

De préférence le contre ion Z⁻ des monomères de formule (II) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (II) pour laquelle, encore plus préférentiellement, m et n sont égaux à zéro.

Le monomère vinyllactame ou alkylvinyllactame est de préférence un composé de structure (V) : 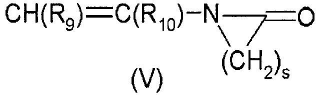 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (V) est la vinylpyrrolidone.

Les polymères poly(vinyllactame) selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (V),
b)-un monomère de formule (II) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (III) dans laquelle R₃ et R₄ désignent, indépendamment l'un de l'autre, un atome d'hydrogène ou un radical alkyle en C₁-C₅.

Encore plus préférentiellement, on met en oeuvre des terpolymères comprenant, en poids, 40 à 95% de monomère (a), 0,1 à 55% de monomère (c) et 0,25 à 50% de monomère (b).

De tels polymères sont notamment décrits dans la demande de brevet WO 00/68282 dont le contenu fait partie intégrante de l'invention.

Comme polymères poly(vinyllactame) selon l'invention, on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide / tosylate ou chlorure de lauryldiméthylméthacrylamidopropylammonium. Le terpolymère vinylpyrrolidone / diméthylaminopropyl méthacrylamide /chlorure de lauryl diméthylméthacrylamidopropylammonium est proposé dans l'eau à 20% par la Société ISP sous la dénomination STYLEZE W20.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone / hexadécène) vendu par la société I.S.P.
- les produits ANTARON V220® ou GANEX V220® (copolymère vinylpyrrolidone / eicosène) vendu par la société I.S.P.

Parmi les dérivés de polyacides insaturés associatifs on peut citer ceux comportant au moins un motif hydrophile de type acide carboxylique insaturé oléfinique, et au moins un motif hydrophobe de type ester d'alkyl (C₁₀-C₃₀) d'acide carboxylique insaturé.

Ces polymères sont notamment choisis parmi ceux dont le motif hydrophile de type acide carboxylique insaturé oléfinique correspond au monomère de formule (VI) suivante : 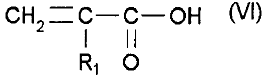 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (VII) suivante : 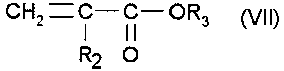 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
(i) essentiellement de l'acide acrylique,
(ii) un ester de formule (VII) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C₁₀-C₃₀ (motif hydrophobe), et de 0 à 6% en poids de monomère polymérisable réticulant, ou bien ceux constitués de 98 à 96% en poids d'acide acrylique (motif hydrophile), de 1 à 4% en poids d'acrylate d'alkyles en C₁₀-C₃₀ (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Parmi les dérivés de polyacides insaturés associatifs on peut aussi citer ceux comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemples de ce type de composés, on peut citer l'ACULYN 22® vendu par la société ROHM et HAAS, qui est un terpolymère acide méthacrylique/acrylate d'éthyle/méthacrylate de stéaryle oxyalkyléné.

La concentration du ou des polymères associatifs présents dans la composition selon l'invention peut varier entre 0,01 et 10% en poids, plus particulièrement entre 0,1 à 5% en poids, par rapport au poids de la composition.

Au cas où la composition est prête à l'emploi, c'est-à-dire qu'elle comprend en outre au moins un agent oxydant, la concentration du ou des polymères associatifs présents dans ladite composition prête à l'emploi, représente 0,0025 à 10% en poids, plus particulièrement 0,025 à 10% en poids, par rapport au poids total de la composition prête à l'emploi.

La composition selon l'invention comprend de plus, à titre d'élément constitutif essentiel, au moins un colorant fluorescent particulier.

Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

Par ailleurs, selon une caractéristique de l'invention, la composition ne comprend pas, à titre de colorant fluorescent, un 2-[2-(4-dialkylamino)phényl éthényl]-1 alkyl pyridinium dans lequel le radical alkyle du moyau pyridinium représente un radical méthyle, éthyle, celui du noyau benzénique représente un radical méthyle, et dans lequel le contre ion est un halogénure.

Conformément à un mode de réalisation encore plus particulier de l'invention, la composition ne comprend pas, à titre de colorant fluorescent, de composé choisi parmi les colorants fluorescents hétérocycliques monocationiques azoiques, azométhiniques ou méthiniques.

Les colorants fluorescents utilisés de préférence selon la présente invention sont des colorants dans la gamme des orangés.

De préférence, les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres.

Certains des colorants fluorescents selon la présente invention sont des composés connus en eux-mêmes.

Les colorants fluorescents mis en oeuvre sont choisis parmi
les colorants fluorescents appartenant aux familles suivantes : les naphtalimides ; les colorants fluorescents polycationiques de type azométhinique, ou méthinique, 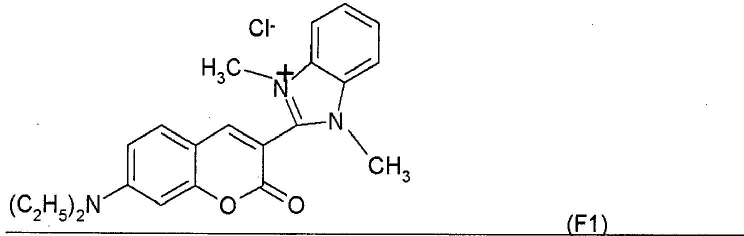 et 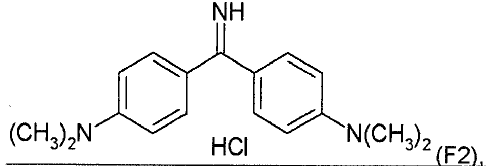 seuls ou en mélanges.

Plus particulièrement, on peut citer parmi eux :
- le Jaune Brilliant B6GL commercialisé par la société SANDOZ et de structure suivante : 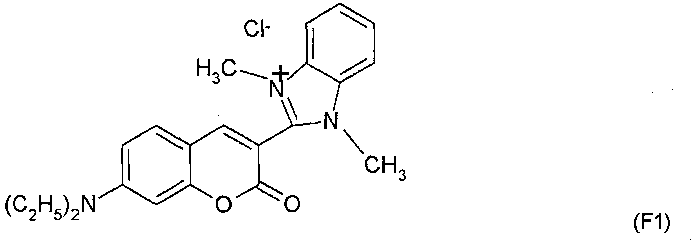
- le Basic Yellow 2, ou Auramine O commercialisé par les sociétés PROLABO, ALDRICH ou CARLO ERBA et de structure suivante : 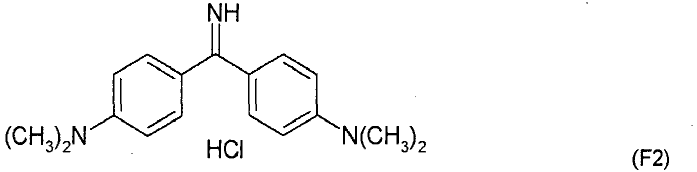 monochlorhydrate de 4,4'-(imidocarbonyl)bis(N,N-diméthylaniline) - CAS number 2465-27-2.

On peut aussi citer les composés de formule suivante : 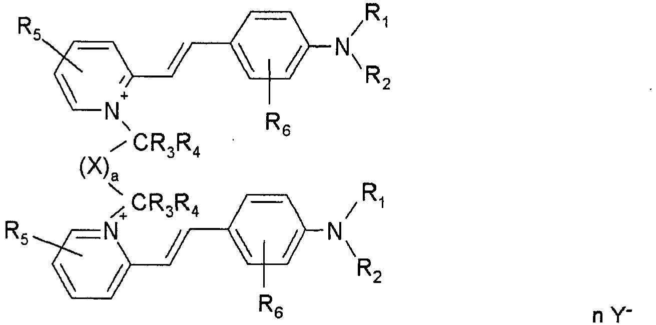 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

Rappelons que les termes hétéroatomes, représentent un atome d'oxygène ou d'azote. Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).

En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; - CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.

De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 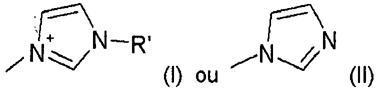 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.

Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.

Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.

De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R67-N(CH3)2 ou -R₆₇-N⁺(CH₃)₃ ou -R₆₇-N⁺(CH₂CH₃)₃ avec R₆₇ représentant un radical alkyle en C₁-C₂.

Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.

En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.

Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.

Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).

Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.

Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re-avec Rc représentant un radical alkyle en C₂-C₉, Rd représentant un atome d'hydrogène, un radical alkyle en C₁-C₂ et Re représentant un radical alkyle en C₁-C₆ ; - Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 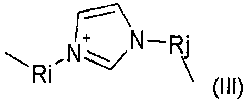 dans laquelle Ri et Rj, identiques ou non, représentent un radical alkyle linéaire en C₁-C₆ ;
X peut de même être choisi parmi les radicaux divalents dérivés de triazine suivants :

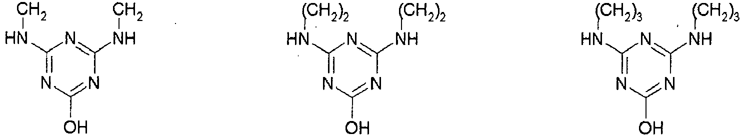

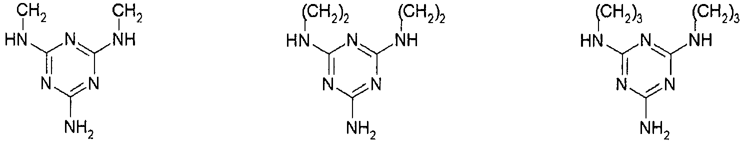

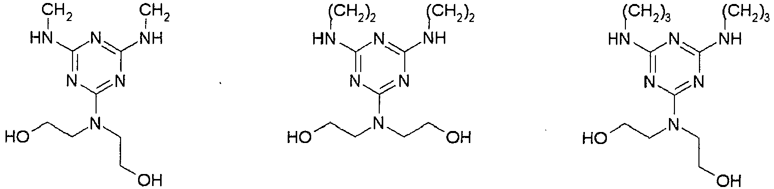

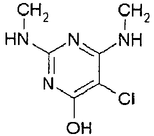

Selon une autre possibilité, X peut représenter les radicaux divalents aromatiques suivants :

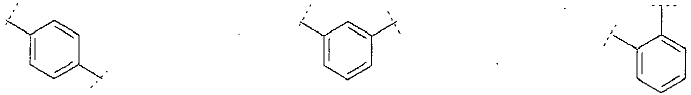

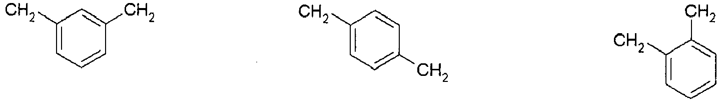

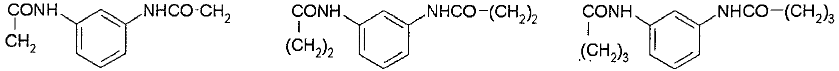

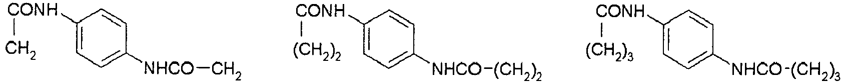

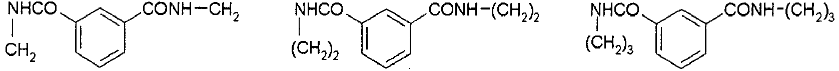

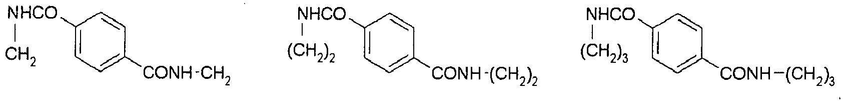

Dans la formule générale de ces composés fluorescents, Y⁻ représente un anion organique ou minéral. S'il y a plusieurs anions Y⁻, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.

Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.

De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore, ou les groupements de type tolylsulfonyle ou méthylesulfonyle.

Cette première étape peut avoir lieu en présence d'un solvant, bien qu'il ne soit pas obligatoire, comme par exemple le diméthylformamide.

Le nombre de moles d'α-picoline est en général voisin de 2 pour une mole de réactif comprenant les groupes partant.

En outre, la réaction est habituellement mise en oeuvre au reflux du réactif et/ou du solvant s'il est présent.

Le produit issu de cette première étape est ensuite contacté avec un aldéhyde correspondant de formule suivante : 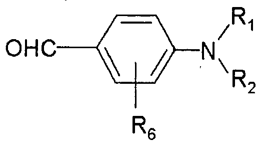 dans laquelle R₁, R₂ et R₆ ont les mêmes significations que précédemment indiquées. Là encore, la réaction peut être effectuée en présence d'un solvant approprié, de préférence au reflux.

Il est à noter que les radicaux R₁ et R₂ de l'aldéhyde peuvent avoir la signification indiquée dans la formule générale détaillée auparavant.

Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Le ou les colorants fluorescents présents dans la composition selon l'invention représentent avantageusement de 0,01 à 20 % en poids, plus particulièrement de 0,05 à 10 % en poids, et de préférence de 0,1 à 5% en poids, du poids total de la composition.

Le milieu cosmétiquement acceptable est généralement constitué par de l'eau ou par un mélange d'eau et d'un ou plusieurs solvants organiques usuels.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition conforme à l'invention est généralement compris entre 3 et 12 environ, et de préférence entre 5 et 11 environ.

II peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 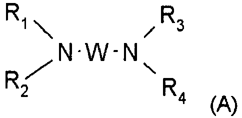 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

Selon un mode de réalisation particulier de l'invention, la composition peut, comprendre, en plus du ou des colorants fluorescents, un ou plusieurs colorants directs additionnels non fluorescents de nature non ionique, cationique ou anionique, qui peuvent par exemple être choisis parmi les colorants benzéniques nitrés.

Conviennent notamment les colorants directs benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène,
- le 1-(β-aminoéthyl)amino-2-nitro-4-(β-hydroxyéthyloxy) benzène,
- le 1-(β,γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition conforme à l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants azoïques, les colorants anthraquinoniques, naphtoquinoniques ou benzoquinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP-A-0 714 954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-(β-hydroxyéthyl)amino 2-nitrobenzène,
- le 1-(β-hydroxyéthyl)amino 4-(N-éthyl, N-(β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 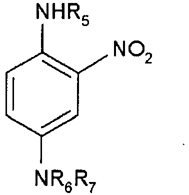 dans laquelle :
   - R₆ représente un radical alkyle en C₁-C₄, un radical β-hydroxyéthyle ou β-hydroxypropyle ou γ-hydroxypropyle ;
   - R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans FR 2 692 572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

Lorsqu'elle est destinée à la coloration d'oxydation, la composition conforme à l'invention comprend, en plus du ou des colorants fluorescents, au moins une base d'oxydation choisie parmi les bases d'oxydation classiquement utilisées pour la coloration d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent de préférence de 0,0005 à 12 % en poids environ du poids total de la composition, et encore plus préférentiellement de 0,005 à 6 % en poids environ de ce poids.

Lorsqu'elle est destinée à la coloration d'oxydation, la composition conforme à l'invention peut également comprendre, en plus des colorants fluorescents et des bases d'oxydation, au moins un coupleur de façon à modifier ou à enrichir en reflets les nuances obtenues en mettant en oeuvre les colorants fluorescents et la ou les bases d'oxydation.

Les coupleurs utilisables dans la composition conforme à l'invention peuvent être choisis parmi les coupleurs utilisés de façon classique en coloration d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, te 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent de préférence de 0,0001 à 10 % en poids, et encore plus préférentiellement de 0,005 à 5 % en poids, du poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.

Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (VIII).

La composition conforme à l'invention peut également comprendre divers adjuvants utilisés classiquement, tels que des agents tensioactifs anioniques, cationiques, non ioniques, amphotères, zwittérioniques ou leurs mélanges, des polymères anioniques, cationiques, non ioniques, amphotères, zwittérioniques autres que ceux de l'invention ou leurs mélanges, des agents épaississants minéraux, des agents antioxydants, des agents de pénétration, des agents séquestrants, des parfums, des tampons, des agents dispersants, des agents de conditionnement tels que par exemple des cations, des silicones volatiles ou non volatiles, modifiées ou non modifiées, des agents filmogènes, des céramides, des agents conservateurs, des agents stabilisants, des agents opacifiants.

On pourra également ajouter des polymères épaississants organiques non associatifs.

Lorsqu'un ou plusieurs agents tensioactifs sont présents, de préférence de type non ionique, anionique ou encore amphotère, leur teneur représente de 0,01 à 30 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention, la composition se trouve sous la forme d'un shampooing colorant et éclaircissant comprenant dans un milieu aqueux cosmétiquement acceptable.

Dans la composition selon l'invention, lorsqu'une ou plusieurs bases d'oxydation sont utilisées, éventuellement en présence d'un ou plusieurs coupleurs, ou lorsque le ou les colorants fluorescents sont utilisés dans le cadre d'une coloration directe éclaircissante, alors la composition conforme à l'invention peut en outre renfermer au moins un agent oxydant.

L'agent oxydant peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

L'invention a également pour objet l'utilisation d'une composition pour colorer avec un effet éclaircissant les matières kératiniques humaines, comprenant dans un milieu cosmétiquement acceptable, au moins un colorant fluorescent soluble dans ledit milieu et au moins un polymère associatif choisi parmi les dérivés de polyuréthanes associatifs, les dérivés de celluloses associatifs, les dérivés de polyvinyllactames associatifs et les dérivés de polyacides insaturés associatifs ledit colorant fluorescent est choisi parmi les colorants fluorescents appartenant aux familles suivantes : les naphtalimides ; les colorants fluorescents polycationiques de type azométhinique, ou méthinique, 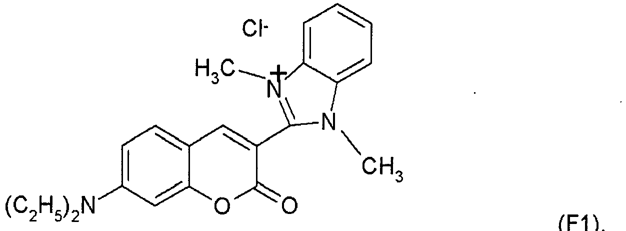 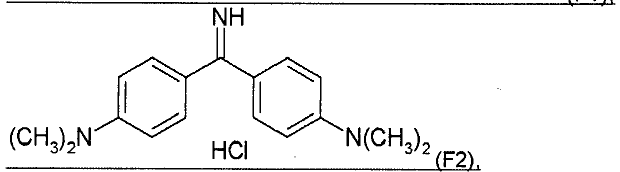 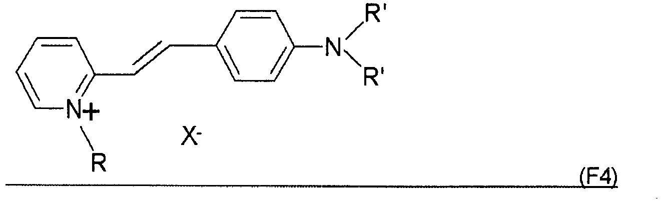 formule dans laquelle R représente un radical méthyle ou éthyle; R' représente un radical méthyle, X- un anion du type chlorure, iodure, sulfate, méthosulfate, acétate, perchlorate, seuls ou en mélanges.

A titre de composés plus particuliers, on peut citer les composés de formules F1, F2 et F3 déjà détaillées auparavant.

On peut de même utiliser les composés de structure (F4) suivante : 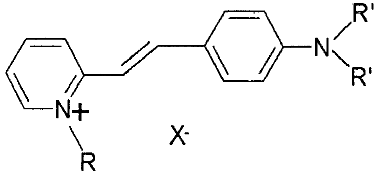 formule dans laquelle R représente un radical méthyle ou éthyle; R' représente un radical méthyle, X- un anion du type chlorure, iodure, sulfate, méthosulfate, acétate, perchlorate. A titre d'exemple de composé de ce type on peut citer le Photosensitiving Dye NK-557 commercialisé par la société UBICHEM, pour lequel R représente un radical éthyle, R' un radical méthyle et X- un iodure.

Tout ce qui a été décrit auparavant sur les natures et teneurs des divers additifs présents dans la composition restent valable et en sera pas repris dans cette partie.

Selon la présente invention, on entend par matières kératiniques humaines, la peau, les cheveux, les ongles, les cils, et les sourcils, et plus particulièrement la peau foncée et les cheveux pigmentés ou colorés artificiellement.

Au sens de l'invention, on entend par peau foncée, une peau dont la luminance L* chiffrée dans le système C.I.E.L. L*a*b* est inférieure ou égale à 45 et de préférence inférieure ou égale à 40, sachant par ailleurs que L*=0 équivaut au noir et L*=100 au blanc. Les types de peau correspondant à cette luminance sont la peau africaine, la peau afro-américaine, la peau hispano-américaine, la peau indienne et la peau maghrébine.

Au sens de l'invention, on entend par cheveux pigmentés ou colorés artificiellement, des cheveux dont la hauteur de ton est inférieure ou égale à 6 (blond foncé) et de préférence inférieure ou égale à 4 (châtain).

L'éclaircissement des cheveux est évalué par la 'hauteur de ton" qui caractérise le degré ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.

Les hauteurs de ton s'échelonnent de 1 (noir) à 10 (blond clair), une unité correspondant à un ton ; plus le chiffre est élevé et plus la nuance est claire.

Un autre objet de la présente invention concerne donc un procédé de coloration avec effet éclaircissant de fibres kératiniques humaines consistant à mettre en oeuvre les étapes suivantes :
a) on applique sur les fibres kératiniques, pendant un temps suffisant pour développer la coloration et l'éclaircissement désirés, la composition selon l'invention,
b) on rince éventuellement lesdites fibres,
c) éventuellement on lave au shampooing et on rince lesdites fibres,
d) on sèche ou on laisse sécher les fibres.

La présente invention a en outre pour objet un procédé pour colorer avec un effet éclaircissant une peau foncée dans lequel on applique sur la peau, la composition qui vient d'être décrite, puis à sécher ou laisser sécher la peau.

Tout ce qui a été précédemment décrit concernant les divers éléments constitutifs de la composition reste valable et l'on pourra s'y reporter.

Notamment, les procédés selon l'invention sont appropriés pour traiter des fibres kératiniques humaines, et notamment les cheveux, pigmentées ou colorées artificiellement, ou encore de la peau foncée.

Plus particulièrement, les fibres qui peuvent être avantageusement traitées par le procédé selon l'invention, présentent une hauteur de ton est inférieure ou égale à 6 (blond foncé) et de préférence inférieure ou égale à 4 (châtain).

De plus, une peau foncée susceptible d'être traitée conformément à l'invention, présente une luminance L*, chiffrée dans le système C.I.E.L. L*a*b*, inférieure ou égale à 45 et de préférence inférieure ou égale à 40.

Selon un premier mode de réalisation de l'invention, le procédé de coloration avec effet éclaircissant des fibres est mis en oeuvre avec une composition ne comprenant pas de colorants d'oxydation ni de coupleur et en l'absence d'agent oxydant.

Selon un deuxième mode de réalisation de l'invention, le procédé de coloration avec effet éclaircissant des fibres est mis en oeuvre avec une composition ne comprenant pas de colorants d'oxydation ni de coupleur, mais en présence d'agent(s) oxydant(s).

Selon une première variante de ces procédés de coloration conformes à l'invention, on applique sur les fibres, et notamment les cheveux, au moins une composition telle que définie précédemment, pendant un temps suffisant pour développer la coloration et l'éclaircissement désirés, après quoi on rince, on lave éventuellement au shampooing, on rince à nouveau et on sèche.

Selon une deuxième variante de ces procédés de coloration conformes à l'invention, on applique sur les fibres, et notamment les cheveux au moins une composition telle que définie précédemment sans rinçage final.

Selon une troisième variante de procédé de coloration conforme à l'invention, le procédé de coloration comporte une étape préliminaire consistant à stocker sous forme séparée, d'une part, une composition selon l'invention comprenant, outre le colorant fluoerscent et le polymère associatif, éventuellement au moins une base d'oxydation et/ou un coupleur et, d'autre part, une composition comprenant, dans un milieu cosmétiquement acceptable, au moins un agent oxydant, puis à procéder à leur mélange au moment de l'emploi avant d'appliquer ce mélange sur les fibres kératiniques, et notamment les cheveux, pendant un temps suffisant pour développer la coloration désirée, après quoi on rince les fibres, on les lave éventuellement au shampooing, on les rince à nouveau et on les sèche.

Le temps nécessaire au développement de la coloration et à l'obtention de l'effet éclaircissant sur les fibres, notamment les cheveux, est d'environ 5 à 60 minutes et plus particulièrement d'environ 5 à 40 minutes.

La température nécessaire au développement de la coloration et à l'obtention de l'effet éclaircissant est généralement comprise entre la température ambiante (15 à 25°C) et 80°C et plus particulièrement entre 15 et 40°C.

Il est aussi ici décrit un dispositif à plusieurs compartiments pour la coloration avec effet éclaircissant des fibres kératiniques et notamment des cheveux, comprenant au moins un compartiment renfermant une composition selon l'invention, et au moins un autre compartiment renfermant une composition comprenant au moins un agent oxydant. Ce dispositif peut être équipé d'un moyen permettant de délivrer sur les fibres le mélange souhaité, tel que les dispositifs décrits dans le brevet FR 2 586 913.

Il est à noter que la composition selon l'invention, si elle est utilisée pour traiter des fibres kératiniques, telles que des cheveux châtains par exemple, permet d'atteindre les résultats suivants :
Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueur d'onde allant de 500 à 700 nanomètres, est supérieure à celle correspondant aux cheveux non traités.

Cela signifie que, dans la gamme de longueur d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.

Il est précisé toutefois qu'il peut exister dans la gamme de longueur d'onde allant de 500 à 700 nanomètres, et de préférence de 540 à 700 nanomètres, une ou plusieurs plages où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

En outre, et de préférence, la composition selon l'invention est susceptible d'éclaircir les cheveux et la peau dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des fibres kératiniques châtain, plus particulièrement des cheveux, à raison de 10 grammes de composition pour 1 gramme de fibres châtain. La composition est étalée de façon à recouvrir l'ensemble des fibres. On laisse la composition agir pendant 20 minutes à température ambiante (20 à 25°C). Les fibres sont ensuite rincées à l'eau puis lavées avec un shampooing à base de lauryléther sulfate. Elles sont ensuite séchées. On mesure alors les caractéristiques spectrocolorimétriques des fibres pour en déterminer les coordonnées L*a*b*.

Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et -b* est bleu) ; des valeurs proches de zéro pour a* et b* correspondent à des nuances grises.

Les exemples qui suivent sont destinés à illustrer l'invention sans pour autant en limiter la portée.

### EXEMPLES

### Composé fluorescent

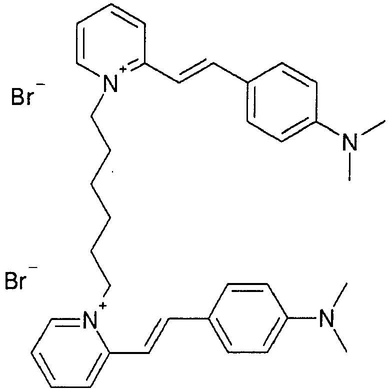

On fait réagir 93 g de 2-picoline avec 120g de 1,6 dibromohexane dans le diméthylformamide à 110°C pendant 5 heures.

On récupère le produit précipité, et on le filtre.

On solubilise 109 g du produit obtenu précédemment dans du méthanol et l'on ajoute 82,82 g de p-diméthylaminobenzaldéhyde en deux fois, en présence de pyrrolidine.

On laisse ensuite pendant 30 minutes.

On récupère le produit sous forme précipitée.
Analyse par spectroscopie de masse : 266.
Analyse élémentaire : C : 62,43 % ; H : 6,40 % ; Br : 23,07 % ; N : 8,09 %.

La formule est la suivante C₃₆H₄₄N₄.2Br.

### Compositions

| **Composition** | **1** | **2** |
|---|---|---|
| Composé fluorescent | 1% | 1% |
| ACULYN 44 (*) | 0,5% | - |
| STYLEZE W20 (**) | - | 0,5% |
| Eau distillée | qsp. 100% | qsp. 100% |

| | | |
|---|---|---|
| Les pourcentages sont exprimés en poids de matière active. (*) dérivé de polyuréthane associatif (**) dérivé de polyvinyllactame associatif | | |

### Coloration

La composition est appliquée sur une mèche de cheveux châtains naturels (hauteur de ton 4) avec un temps de pose de 20 minutes.

Les mèches sont ensuite rincées et un séchées au casque pendant 30 minutes.

On observe sur la mèche, un net effet d'éclaircissement.

## Revendications

1. Composition, **caractérisée en ce qu'**elle comprend, dans un milieu cosmétiquement acceptable, au moins un colorant fluorescent soluble dans ledit milieu, et au moins un polymère associatif choisi parmi les dérivés de polyuréthanes associatifs, les dérivés de celluloses associatifs, les dérivés de polyvinyllactames associatifs et les dérivés de polyacides insaturés associatifs ; la composition ne comprenant pas, à titre d'agent fluorescent, du 2-[2-(4-dialkylamino)phényl éthényl]-1 alkyl pyridinium dans lequel le radical alkyle du noyau pyridinium représente un radical méthyle, éthyle, celui du noyau benzénique représente un radical méthyle et dans lequel le contre ion est un halogénure,
**caractérisée en ce que** le colorant fluorescent est choisi parmi les colorants fluorescents appartenant aux familles suivantes : les naphtalimides ; les colorants fluorescents polycationiques de type azométhinique ou méthinique, et

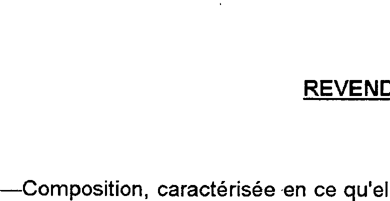

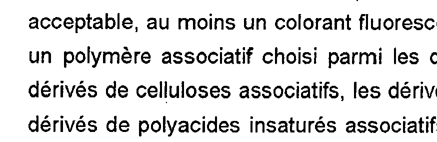

2. Composition selon la revendication 1, **caractérisée en ce que** le polymère associatif est de nature cationique, non ionique, anionique ou amphotère.

3. Composition selon l'une des revendications 1 ou 2, **caractérisée en ce que** le polyuréthane associatif est anionique et comprend au moins un motif provenant d'un monomère du type acide carboxylique à insaturation α,β-monoéthylénique.

4. Composition selon l'une des revendications 1 ou 2, **caractérisée en ce que** le polyuréthane associatif est cationique et correspond à la formule générale (Ia) suivante :
R-X-(P)ₙ-[L-(Y)ₘ]ᵣ-L'-(P')ₚ-X'-R' (Ia)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe.

5. Composition selon l'une des revendications 1 ou 2, **caractérisée en ce que** le polyuréthane associatif est un polyuréthane polyéther non ionique.

6. Composition selon l'une des revendications 1 ou 2, **caractérisée en ce que** le dérivé de cellulose associatif est cationique et est choisi parmi les celluloses ou les hydroxyéthylcelluloses quaternisées par un groupement hydrophobe.

7. Composition selon l'une des revendications 1 ou 2, **caractérisée en ce que** le dérivé de cellulose associatif est non ionique et est choisi parmi les hydroxyéthyl celluloses modifiées par un groupement hydrophobe.

8. Composition selon l'une des revendications 1 ou 2, **caractérisée en ce que** le dérivé de polyvinyllactame associatif est cationique et comprend :
a) au moins un monomère de type vinyl lactame ou alkylvinyllactame;
b) au moins un monomère de structures (II) ou (III) suivantes : 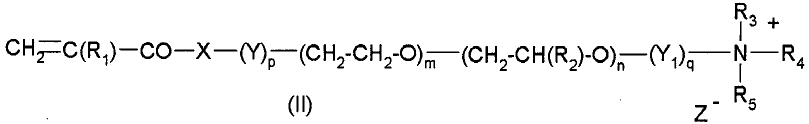 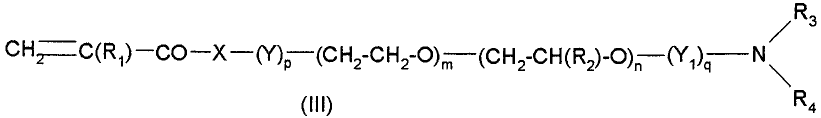 dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (IV):
-(Y₂)ᵣ-(CH₂-CH(R₇)-O)ₓ-R₈ (IV)
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
si m ou n sont égaux à zéro, alors p ou q est égal à 0.

9. Composition selon l'une des revendications 1 ou 2, **caractérisée en ce que** le dérivé de polyvinyllactame associatif est non ionique et est choisi parmi les copolymères de vinyl pyrrolidone et d'au moins un monomère hydrophobe à chaîne grasse.

10. Composition selon l'une des revendications 1 ou 2, **caractérisée en ce que** le dérivé de polyacide insaturé associatif est un polymère anionique comportant au moins un motif hydrophile de type acide carboxylique insaturé oléfinique, et au moins un motif hydrophobe de type ester d'alkyl (C₁₀-C₃₀) d'acide carboxylique insaturé.

11. Composition selon l'une des revendications 1 ou 2, **caractérisée en ce que** le dérivé de polyacide insaturé associatif est un polymère anionique comportant parmi ses monomères un acide carboxylique à insaturation α,β-monoéthylénique et un ester d'acide carboxylique à insaturation α,β-monoéthylénique et d'un alcool gras oxyalkyléné.

12. Composition selon l'une des revendications 10 ou 11, **caractérisée en ce que** l'acide carboxylique est l'acide acrylique ou l'acide méthacrylique.

13. Composition selon l'une quelconque des revendications 1 à 12, **caractérisée en ce que** la teneur du ou des polymères associatifs varie de 0,01 à 10% en poids, plus particulièrement de 0,1 à 5% en poids, du poids total de la composition.

14. Composition selon l'une quelconque des revendications précédentes, **caractérisée en ce que** le colorant fluorescent, éventuellement neutralisé, est soluble dans le milieu cosmétique à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

15. Composition selon l'une quelconque des revendications précédentes, **caractérisée en ce que** le colorant fluorescent est un colorant dans la gamme des orangés.

16. Composition selon l'une quelconque des revendications précédentes, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

17. Composition selon l'une quelconque des revendications précédentes, **caractérisée en ce que** le colorant fluorescent est choisi dans le groupe formé par les colorants de structures suivantes : 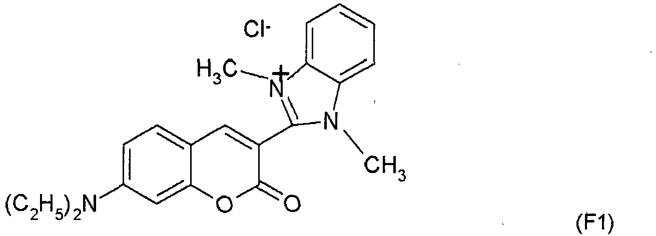 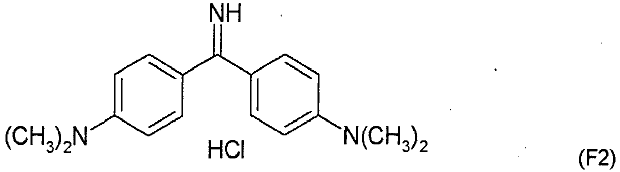 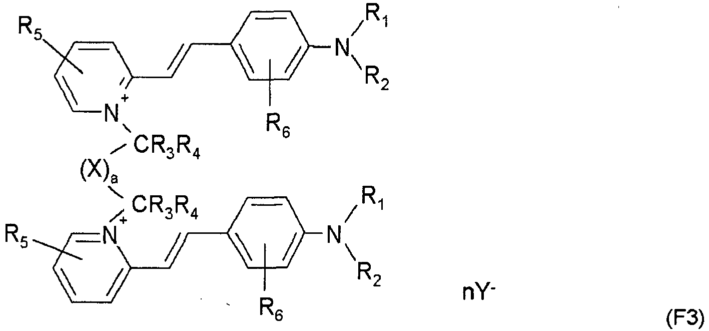 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ;
un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

18. Composition selon l'une quelconque des revendications précédentes, **caractérisée en ce que** le ou les colorants fluorescents sont présents dans une concentration pondérale comprise entre 0,01 et 20% en poids, plus particulièrement comprise entre 0,05 à 10% en poids, de préférence comprise entre 0,1 à 5% en poids, par rapport au poids total de la composition.

19. Composition selon l'une quelconque des revendications précédentes, **caractérisée en ce qu'**elle comprend en outre au moins un colorant direct additionnel non fluorescent de nature non ionique, cationique ou anionique.

20. Composition selon la revendication 19, **caractérisée par le fait que** les colorants directs additionnels sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

21. Composition selon l'une des revendications 19 ou 20, **caractérisée en ce que** le ou les colorants directs additionnels représentent de 0,0005 à 12 % en poids, de préférence de 0,005 à 6 % en poids, du poids total de la composition.

22. Composition selon l'une quelconque des revendications précédentes, **caractérisée en ce qu'**elle se présente sous la forme d'un shampooing éclaircissant et colorant.

23. Composition selon l'une quelconque des revendications précédentes, **caractérisée en ce qu'**elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

24. Composition selon la revendication 23, **caractérisée en ce que** la ou les bases d'oxydation représentent 0,0005 à 12 % en poids, plus particulièrement 0,005 à 6 % en poids, du poids total de la composition.

25. Composition selon l'une quelconque des revendications 23 ou 24, **caractérisée en ce qu'**elle comprend au moins un coupleur choisi parmi les métaphénylène diamines, les méta-aminophénols, les métadiphénols et les coupleurs hétérocycliques ou leurs sels d'addition avec un acide ou avec un agent alcalin.

26. Composition selon la revendication 25, **caractérisée en ce que** le ou les coupleurs représentent de 0,0001 à 10 % en poids, plus particulièrement 0,005 à 5 % en poids, du poids total de la composition.

27. Composition prête à l'emploi, **caractérisée en ce qu'**elle comprend, la composition selon l'une des revendications 1 à 26, et au moins un agent oxydant.

28. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

29. Procédé pour colorer avec un effet éclaircissant les fibres kératiniques humaines, **caractérisé en ce que** l'on met en oeuvre les étapes suivantes :
a) on applique sur lesdites fibres une composition telle que définie selon l'une quelconque des revendications 1 à 28, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

30. Procédé selon la revendication 29, **caractérisé en ce qu'**il comporte une étape préliminaire consistant à stocker sous forme séparée, d'une part, une composition selon l'une des revendications 1 à 21 et 23 à 26, et d'autre part, une composition renfermant, dans un milieu cosmétiquement acceptable, au moins un agent oxydant, puis à procéder à leur mélange au moment de l'emploi avant d'appliquer ce mélange sur les fibres pendant un temps suffisant pour développer la coloration désirée, après quoi on les rince, on les lave éventuellement au shampooing, on les rince à nouveau et on les sèche.

31. Procédé selon l'une quelconque des revendications 29 ou 30, **caractérisé par le fait que** la composition est appliquée sur des cheveux présentant une hauteur de ton selon l'échelle définie dans l'ouvrage de Charles Zviak, « Science des traitements capillaires », 1988, aux pages 215 et 278, inférieure ou égale à 6 et de préférence inférieure ou égale à 4.

32. Procédé selon l'une des revendications 29 à 31, **caractérisé en ce que** les fibres kératiniques humaines sont pigmentées ou colorées artificiellement.

33. Procédé pour colorer avec un effet éclaircissant une peau foncée, **caractérisé en ce que** l'on applique sur la peau une composition selon l'une quelconque des revendications 1 à 21, puis on sèche ou on laisse sécher la peau.

34. Utilisation d'une composition comprenant, dans un milieu cosmétiquement acceptable, au moins un colorant fluorescent soluble dans ledit milieu, et au moins un polymère associatif choisi parmi les dérivés de polyuréthanes associatifs, les dérivés de celluloses associatifs, les dérivés de polyvinyllactames associatifs et les dérivés de polyacides insaturés associatifs pour la coloration avec effet éclaircissant des matières kératiniques;
**caractérisée en ce que** le colorant fluorescent est choisi parmi les colorants fluorescents appartenant aux familles suivantes : les naphtalimides ; les colorants fluorescents polycationiques de type azométhinique, ou méthinique, 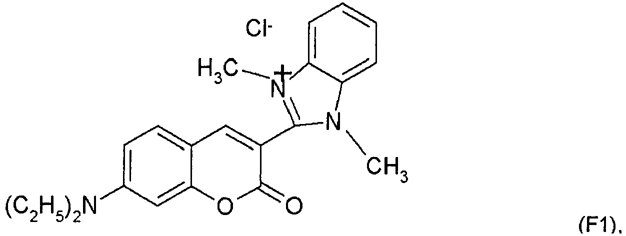 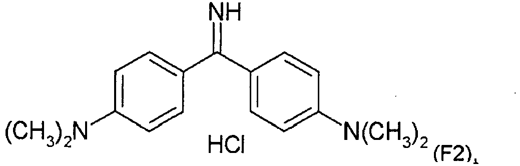 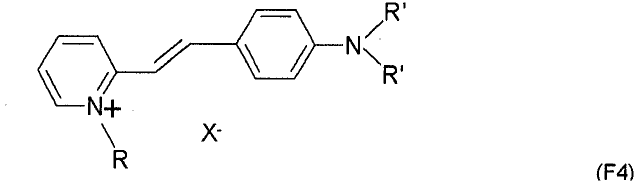 formule dans laquelle R représente un radical méthyle ou éthyle; R' représente un radical méthyle, X- un anion du type chlorure, iodure, sulfate, méthosulfate, acétate, perchlorate, seuls ou en mélanges.

35. Utilisation selon la revendication précédente, **caractérisée en ce que** le colorant fluorescent est un colorant dans la gamme des orangés.

36. Utilisation selon l'une quelconque des revendications 34 ou 35, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

37. Utilisation selon l'une quelconque des revendications 34 à 36, **caractérisée en ce que** le colorant fluorescent est choisi dans le groupe formé par les colorants de structures suivantes : 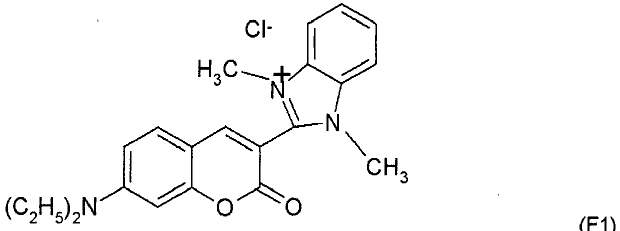 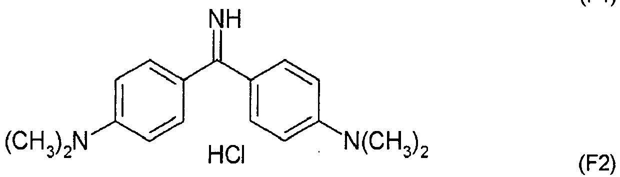 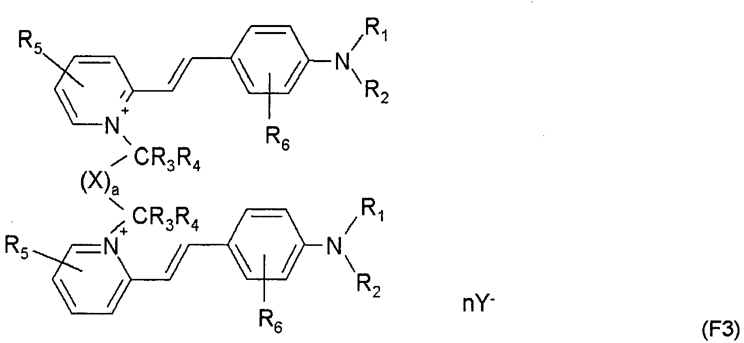 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent ; 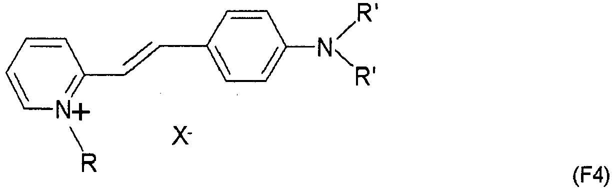 formule dans laquelle R représente un radical méthyle ou éthyle; R' représente un radical méthyle, X- un anion du type chlorure, iodure, sulfate, méthosulfate, acétate, perchlorate.

38. Utilisation selon l'une quelconque des revendications 34 à 37, **caractérisée par le fait que** le ou les colorants fluorescents sont présents dans une concentration pondérale allant de 0,01 à 20% en poids, plus particulièrement de 0,05 à 10% en poids, de préférence de 0,1 à 5% en poids, par rapport au poids total de la composition.

39. Utilisation selon l'une des revendications 34 à 38, **caractérisée en ce que** la teneur du ou des polymères associatifs varie de 0,01 à 10% en poids, plus particulièrement de 0,1 à 5% en poids, du poids total de la composition.

40. Utilisation selon l'une des revendications 34 à 39, **caractérisée en ce que** les matières kératiniques sont des fibres kératiniques pigmentées ou colorées artificiellement, en particulier des cheveux, ou de la peau foncée.

41. Utilisation selon la revendication 40, **caractérisée par le fait que** les cheveux présentent une hauteur de ton selon l'échelle définie dans l'ouvrage de Charles Zviak. « Science des traitements capillaires », 1988, aux pages 215 et 278, inférieure ou égale à 6 et de préférence inférieure ou égale à 4.

## Patentansprüche

1. Zusammensetzung, **dadurch gekennzeichnet, dass** sie in einem kosmetisch akzeptablen Medium mindestens einen in dem Medium löslichen fluoreszierenden Farbstoff und mindestens ein assoziatives Polymer, ausgewählt aus assoziativen Polyurethanderivaten, assoziativen Cellulosederivaten, assoziativen Polyvinyllactamderivaten und assoziativen Derivaten ungesättigter Polysäuren, umfasst; wobei die Zusammensetzung als fluoreszierende Substanz nicht 2-[2-(4-Dialkylamino)phenylethenyl]-1-alkylpyridinium umfasst, worin der Alkylrest des Pyridiniumkerns einen Methyl-, Ethylrest darstellt, derjenige des Benzolkerns einen Methylrest darstellt und worin das Gegenion ein Halogenid ist,
**dadurch gekennzeichnet, dass** der fluoreszierende Farbstoff aus fluoreszierenden Farbstoffen, die zu den folgenden Familien gehören: Naphthalimiden; polykationischen fluoreszierenden Farbstoffen des Azomethin- oder Methintyps und 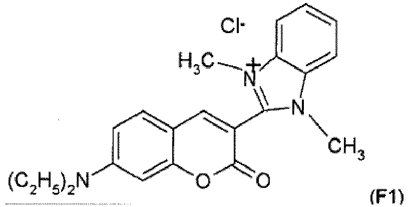 und 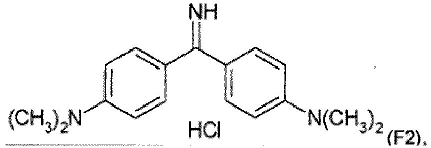 allein oder in Gemischen ausgewählt ist.

2. Zusammensetzung nach Anspruch 1, **dadurch gekennzeichnet, dass** das assoziative Polymer kationischer, nichtionischer, anionischer oder amphoterer Natur ist.

3. Zusammensetzung nach einem der Ansprüche 1 oder 2, **dadurch gekennzeichnet, dass** das assoziative Polyurethan anionisch ist und mindestens ein Motiv umfasst, das von einem Monomer des Typs einer Carbonsäure mit α,β-monoethylenischer Ungesättigtheit stammt.

4. Zusammensetzung nach einem der Ansprüche 1 oder 2, **dadurch gekennzeichnet, dass** das assoziative Polyurethan kationisch ist und der folgenden allgemeinen Formel (Ia) entspricht:
R-X-(P)ₙ-[L-(Y)ₘ]ᵣ-L'-(P')ₚ-X'-R' (Ia),
worin:
R und R', die gleich oder verschieden sind, eine hydrophobe Gruppe oder ein Wasserstoffatom darstellen;
X und X', die gleich oder verschieden sind, eine Gruppe, die eine Aminfunktion trägt, die eine hydrophobe Gruppe trägt oder nicht, oder auch die Gruppe L" darstellen;
L, L' und L", die gleich oder verschieden sind, eine von einem Diisocyanat stammende Gruppe darstellen;
P und P', die gleich oder verschieden sind, eine Gruppe darstellen, die eine Aminofunktion trägt, die eine hydrophobe Gruppe trägt oder nicht;
Y eine hydrophile Gruppe darstellt;
r eine ganze Zahl zwischen 1 und 100, vorzugsweise zwischen 1 und 50 und insbesondere zwischen 1 und 25 ist,
n, m und p jeweils unabhängig voneinander einen Wert zwischen 0 bis 1000 haben;
wobei das Molekül mindestens eine protonierte oder quaternisierte Aminfunktion und mindestens eine hydrophobe Gruppe enthält.

5. Zusammensetzung nach einem der Ansprüche 1 oder 2, **dadurch gekennzeichnet, dass** es sich bei dem assoziativen Polyurethan um ein nichtionisches Polyether-Polyurethan handelt.

6. Zusammensetzung nach einem der Ansprüche 1 oder 2, **dadurch gekennzeichnet, dass** das assoziative Cellulosederivat kationisch ist und aus mit einer hydrophoben Gruppe quaternisierten Cellulosen und Hydroxyethylcellulosen ausgewählt ist.

7. Zusammensetzung nach einem der Ansprüche 1 oder 2, **dadurch gekennzeichnet, dass** das assoziative Cellulosederivat nichtionisch ist und aus mit einer hydrophoben Gruppe modifizierten Hydroxyethylcellulosen ausgewählt ist.

8. Zusammensetzung nach einem der Ansprüche 1 oder 2, **dadurch gekennzeichnet, dass** das assoziative Polyvinyllactamderivat kationisch ist und Folgendes umfasst:
a) mindestens ein Monomer des Vinyllactam- oder Alkylvinyllactamtyps;
b) mindestens ein Monomer der folgenden Strukturen (II) oder (III) : 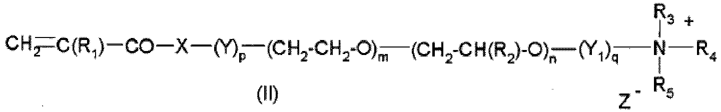 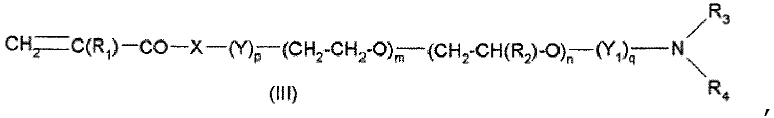 worin:
X für ein Sauerstoffatom oder einen Rest NR₆ steht,
R₁ und R₆ unabhängig voneinander für ein Wasserstoffatom oder einen geraden oder verzweigten C₁-C₅-Alkylrest stehen,
R₂ für einen für geraden oder verzweigten C₁-C₄-Alkylrest steht,
R₃, R₄ und R₅ unabhängig voneinander für ein Wasserstoffatom, einen geraden oder verzweigten C₁-C₃₀-Alkylrest oder einen Rest der Formel (IV):
-(Y₂)ᵣ-(CH₂-CH(R₇)-O)ₓ-R₈ (IV)
stehen,
Y, Y₁ und Y₂ unabhängig voneinander für einen geraden oder verzweigten C₂-C₁₆-Alkylenrest stehen,
R₇ für ein Wasserstoffatom oder einen geraden oder verzweigten C₁-C₄-Alkylrest oder einen geraden oder verzweigten C₁-C₄-Hydroxyalkylrest steht,
R₈ für ein Wasserstoffatom oder einen geraden oder verzweigten C₁-C₃₀-Alkylrest steht,
p, q und r unabhängig voneinander entweder für den Wert null oder den Wert 1 stehen,
m und n unabhängig voneinander für eine ganze Zahl von 0 bis 100 stehen,
x für eine ganze Zahl von 1 bis 100 steht,
Z für ein organisches oder anorganisches Säureanion steht,
mit der Maßgabe, dass:
- mindestens einer der Substituenten R₃, R₄, R₅ oder R₈ für einen geraden oder verzweigten C₉-C₃₀-Alkylrest steht,
- wenn m oder n verschieden von null ist, q dann gleich 1 ist,
wenn m oder n gleich null sind, p oder q dann 0 ist.

9. Zusammensetzung nach einem der Ansprüche 1 oder 2, **dadurch gekennzeichnet, dass** das assoziative Polyvinyllactamderivat nichtionisch ist und aus Copolymeren von Vinylpyrrolidon und mindestens einem hydrophoben Monomer mit Fettkette ausgewählt ist.

10. Zusammensetzung nach einem der Ansprüche 1 oder 2, **dadurch gekennzeichnet, dass** das assoziative Derivat einer ungesättigten Polysäure ein anionisches Polymer ist, das mindestens ein hydrophiles Motiv des Typs einer ungesättigten olefinischen Carbonsäure und mindestens ein hydrophobes Motiv des Typs eines (C₁₀-C₃₀)Alkylesters einer ungesättigten Carbonsäure trägt.

11. Zusammensetzung nach einem der Ansprüche 1 oder 2, **dadurch gekennzeichnet, dass** das assoziative Derivat einer ungesättigten Polysäure ein anionisches Polymer ist, das unter seinen Monomeren eine Carbonsäure mit α,β-monoethylenischer Ungesättigtheit und einen Ester einer Carbonsäure mit α,β-monoethylenischer Ungesättigtheit und einen alkoxylierten Fettalkohol enthält.

12. Zusammensetzung nach einem der Ansprüche 10 oder 11, **dadurch gekennzeichnet, dass** es sich bei der Carbonsäure um Acrylsäure oder Methacrylsäure handelt.

13. Zusammensetzung nach einem der Ansprüche 1 bis 12, **dadurch gekennzeichnet, dass** der Gehalt des oder der assoziativen Polymer(s/e) von 0,01 bis 10 Gew.-%, ganz besonders von 0,1 bis 5 Gew.-%, bezogen auf das Gesamtgewicht der Zusammensetzung, variiert.

14. Zusammensetzung nach einem der vorhergehenden Ansprüche, **dadurch gekennzeichnet, dass** der gegebenenfalls neutralisierte fluoreszierende Farbstoff in dem kosmetischen Medium zu mindestens 0,001 g/l, ganz besonders mindestens 0,5 g/l, vorzugsweise mindestens 1 g/l und in einer noch stärker bevorzugten Ausführungsform mindestens 5 g/l bei einer Temperatur zwischen 15 und 25°C löslich ist.

15. Zusammensetzung nach einem der vorhergehenden Ansprüche, **dadurch gekennzeichnet, dass** der fluoreszierende Farbstoff ein Farbstoff im Orangebereich ist.

16. Zusammensetzung nach einem der vorhergehenden Ansprüche, **dadurch gekennzeichnet, dass** der fluoreszierende Farbstoff zu einer maximalen Reflexion im Wellenlängenbereich von 500 bis 650 Nanometer und vorzugsweise im Wellenlängenbereich von 550 bis 620 Nanometer führt.

17. Zusammensetzung nach einem der vorhergehenden Ansprüche, **dadurch gekennzeichnet, dass** der fluoreszierende Farbstoff aus der Gruppe ausgewählt ist, die von den Farbstoffen der folgenden Strukturen gebildet wird: 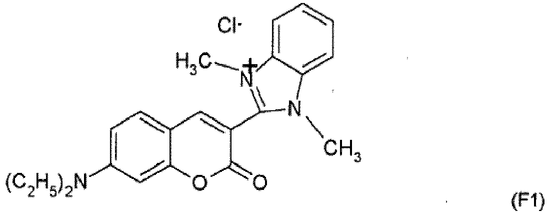 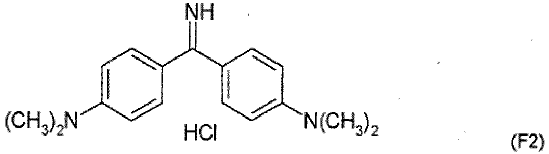 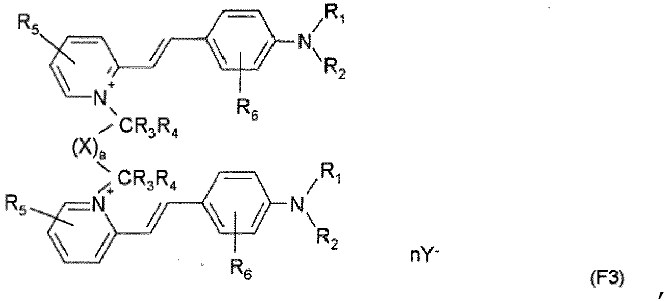 worin:
R₁, R₂, die gleich oder verschieden sind, für Folgendes stehen:
• ein Wasserstoffatom;
• einen geraden oder verzweigten Alkylrest mit 1 bis 10 Kohlenstoffatomen, vorzugsweise 1 bis 4 Kohlenstoffatomen, der gegebenenfalls durch mindestens ein Heteroatom und/oder eine Gruppe, die mindestens ein Heteroatom umfasst, unterbrochen und/oder substituiert ist und/oder mit mindestens einem Halogenatom substituiert ist;
• einen Aryl- oder Arylalkylrest, wobei der Arylrest 6 Kohlenstoffatome besitzt und der Alkylrest 1 bis 4 Kohlenstoffatome besitzt, wobei der Arylrest gegebenenfalls mit einem oder mehreren geraden oder verzweigten Alkylresten mit 1 bis 4 Kohlenstoffatomen substituiert ist, die gegebenenfalls durch mindestens ein Heteroatom und/oder eine Gruppe, die mindestens ein Heteroatom umfasst, unterbrochen und/oder substituiert sind, und/oder mit mindestens einem Halogenatom substituiert ist;
• wobei R₁ und R₂ gegebenenfalls derart verbunden sein können, dass sie mit dem Stickstoffatom einen Heterozyklus bilden, und ein oder mehrere weitere Heteroatome umfassen können, wobei der Heterozyklus gegebenenfalls mit mindestens einem geraden oder verzweigten Alkylrest substituiert ist, der vorzugsweise 1 bis 4 Kohlenstoffatome umfasst und gegebenenfalls durch mindestens ein Heteroatom und/oder eine Gruppe, die mindestens ein Heteroatom umfasst, unterbrochen und/odersubstituiert ist und/oder mit mindestens einem Halogenatom substituiert ist;
• R₁ oder R₂ gegebenenfalls in einen Heterozyklus eingebunden sein können, der das Stickstoffatom und eines der Kohlenstoffatome der Phenylgruppe, die das Stickstoffatom trägt, umfasst;
R₃, R₄, die gleich sind oder nicht, ein Wasserstoffatom, einen Alkylrest mit 1 bis 4 Kohlenstoffatomen darstellen;
R₅, die gleich sind oder nicht, ein Wasserstoffatom, ein Halogenatom, einen geraden oder verzweigten Alkylrest mit 1 bis 4 Kohlenstoffatomen, der gegebenenfalls durch mindestens ein Heteroatom unterbrochen ist, darstellen; R₆, die gleich sind oder nicht, ein Wasserstoffatom; ein Halogenatom; einen geraden oder verzweigten Alkylrest mit 1 bis 4 Kohlenstoffatomen, der gegebenenfalls durch mindestens ein Heteroatom und/oder eine Gruppe, die mindestens ein Heteroatom trägt, substituiert und/oder unterbrochen ist und/oder mit mindestens einem Halogenatom substituiert ist;
X Folgendes darstellt:
• einen geraden oder verzweigten Alkylrest mit 1 bis 14 Kohlenstoffatomen oder einen Alkenylrest mit 2 bis 14 Kohlenstoffatomen, der gegebenenfalls durch mindestens ein Heteroatom und/oder eine Gruppe, die mindestens ein Heteroatom umfasst, unterbrochen und/oder substituiert ist und/oder mit mindestens einem Halogenatom substituiert ist;
• einen 5 oder 6 Ringglieder umfassenden heterozyklischen Rest, der gegebenenfalls mit mindestens einem geraden oder verzweigten Alkylrest mit 1 bis 14 Kohlenstoffatomen, der gegebenenfalls mit mindestens einem Heteroatom substituiert ist; mit mindestens einem geraden oder verzweigten Aminoalkylrest mit 1 bis 4 Kohlenstoffatomen, der gegebenenfalls mit mindestens einem Heteroatom substituiert ist; mit mindestens einem Halogenatom substituiert ist;
• einen aromatischen oder einen kondensierten oder nicht-kondensierten diaromatischen Rest, der durch einen Alkylrest mit 1 bis 4 Kohlenstoffatomen getrennt ist oder nicht, wobei der oder die Arylrest(e) gegebenenfalls mit mindestens einem Halogenatom oder mit mindestens einem Alkylrest, der 1 bis 10 Kohlenstoffatome umfasst, der gegebenenfalls durch mindestens ein Heteroatom und/oder eine Gruppe, die mindestens ein Heteroatom trägt, unterbrochen und/oder substituiert ist, substituiert ist;
• einen Dicarbonylrest;
• wobei die Gruppe X eine oder mehrere kationische Ladungen tragen kann;
a gleich 0 oder 1 ist;
Y⁻, die gleich sind oder nicht, ein organisches oder anorganisches Anion darstellen;
n eine ganze Zahl von mindestens gleich 2 und höchstens gleich der Anzahl an kationischen Ladungen, die in der fluoreszierenden Verbindung vorliegen, ist.

18. Zusammensetzung nach einem der vorhergehenden Ansprüche, **dadurch gekennzeichnet, dass** der oder die fluoreszierende(n) Farbstoff (e) in einer Konzentration, bezogen auf Gewicht, zwischen 0,01 und 20 Gew.-%, insbesondere zwischen 0,05 und 10 Gew.-%, vorzugsweise zwischen 0,1 und 5 Gew.-%, bezogen auf das Gesamtgewicht der Zusammensetzung, vorhanden ist/sind.

19. Zusammensetzung nach einem der vorhergehenden Ansprüche, **dadurch gekennzeichnet, dass** sie außerdem mindestens einen zusätzlichen nicht-fluoreszierenden, nichtionischen, kationischen oder anionischen Direktfarbstoff umfasst.

20. Zusammensetzung nach Anspruch 19, **dadurch gekennzeichnet, dass** die zusätzlichen Direktfarbstoffe aus nitrierten Benzolfarbstoffen, Azo-, Anthrachinon-, Naphthochinon-, Benzochinon-, Phenotiazin-, indigoiden, Xanthen-, Phenanthridinfarbstoffen, Phthalocyaninen sowie den von Triarylmethan stammenden Farbstoffen oder deren Gemischen ausgewählt sind.

21. Zusammensetzung nach einem der Ansprüche 19 oder 20, **dadurch gekennzeichnet, dass** der oder die zusätzliche(n) Direktfarbstoff(e) 0,0005 bis 12 Gew.-%, vorzugsweise 0,005 bis 6 Gew.-%, bezogen auf das Gesamtgewicht der Zusammensetzung, ausmachen.

22. Zusammensetzung nach einem der vorhergehenden Ansprüche, **dadurch gekennzeichnet, dass** sie in Form eines aufhellenden und färbenden Haarwaschmittels vorliegt.

23. Zusammensetzung nach einem der vorhergehenden Ansprüche, **dadurch gekennzeichnet, dass** sie mindestens eine Oxidationsbase umfasst, die aus paraPhenylendiaminen, Bisphenylalkylendiaminen, para-Aminophenolen, ortho-Aminophenolen und heterozyklischen Basen oder deren Additionssalzen mit einer Säure oder mit einer alkalischen Substanz ausgewählt ist.

24. Zusammensetzung nach Anspruch 23, **dadurch gekennzeichnet, dass** die Oxidationsbase(n) 0,0005 bis 12 Gew.-%, insbesondere 0,005 bis 6 Gew.-%, bezogen auf das Gesamtgewicht der Zusammensetzung, ausmachen.

25. Zusammensetzung nach einem der Ansprüche 23 oder 24, **dadurch gekennzeichnet, dass** sie mindestens einen Kuppler umfasst, der aus meta-Phenylendiaminen, meta-Aminophenolen, meta-Diphenolen und heterozyklischen Kupplern oder deren Additionssalzen mit einer Säure oder mit einer alkalischen Substanz ausgewählt ist.

26. Zusammensetzung nach Anspruch 25, **dadurch gekennzeichnet, dass** der oder die Kuppler 0,0001 bis 10 Gew.-%, insbesondere 0,005 bis 5 Gew.-%, bezogen auf das Gesamtgewicht der Zusammensetzung, ausmachen.

27. Gebrauchsfertige Zusammensetzung, **dadurch gekennzeichnet, dass** sie die Zusammensetzung nach einem der Ansprüche 1 bis 26 und mindestens ein Oxidationsmittel umfasst.

28. Zusammensetzung nach dem vorhergehenden Anspruch, **dadurch gekennzeichnet, dass** das Oxidationsmittel aus Wasserstoffperoxid, Harnstoffperoxid, Alkalimetallbromaten, Persalzen, wie Perboraten und Persulfaten, und Enzymen, wie Peroxidasen und Zwei- oder Vier-Elektronen-Oxidoreduktasen, ausgewählt ist.

29. Verfahren zum Färben menschlicher Keratinfasern mit einer aufhellenden Wirkung, **dadurch gekennzeichnet, dass** man die folgenden Schritte durchführt:
a) man bringt auf die Fasern eine Zusammensetzung nach einem der Ansprüche 1 bis 28 für eine Zeit auf, die ausreichend ist, dass sich die gewünschte Färbung und Aufhellung entwickeln,
b) man spült gegebenenfalls die Fasern,
c) gegebenenfalls wäscht man die Fasern mit Haarwaschmittel und spült die Fasern,
d) man trocknet die Fasern oder lässt sie trocknen.

30. Verfahren nach Anspruch 29, **dadurch gekennzeichnet, dass** es einen vorbereiteten Schritt enthält, der daraus besteht, dass man getrennt voneinander einerseits eine Zusammensetzung nach einem der Ansprüche 1 bis 21 und 23 bis 26 und andererseits eine Zusammensetzung, die in einem kosmetisch akzeptablen Medium mindestens ein Oxidationsmittel enthält, aufbewahrt und diese dann zum Zeitpunkt der Verwendung mischt, bevor dieses Gemisch auf die Fasern während einer Zeit aufgebracht wird, die ausreichend ist, dass sich die gewünschte Färbung entwickelt, wonach man sie spült, sie gegebenenfalls mit Haarwaschmittel wäscht, sie nochmals spült und trocknet.

31. Verfahren nach einem der Ansprüche 29 oder 30, **dadurch gekennzeichnet, dass** die Zusammensetzung auf Haare aufgetragen wird, die eine Farbtiefe gemäß der in dem Handbuch von Charles Zviak "Science des traitements capillaires", 1988, auf den Seiten 215 und 278 definierten Skala von weniger als oder gleich 6 und vorzugsweise weniger als oder gleich 4 aufweisen.

32. Verfahren nach einem der Ansprüche 29 bis 31, **dadurch gekennzeichnet, dass** die menschlichen Keratinfasern pigmentiert oder künstlich gefärbt.

33. Verfahren zum Färben von dunkler Haut mit aufhellender Wirkung, **dadurch gekennzeichnet, dass** man auf die Haut eine Zusammensetzung nach einem der Ansprüche 1 bis 21 aufbringt, dann die Haut trocknet oder trocknen lässt.

34. Verwendung einer Zusammensetzung, die in einem kosmetisch akzeptablen Medium mindestens einen in dem Medium löslichen fluoreszierenden Farbstoff und mindestens ein assoziatives Polymer, ausgewählt aus assoziativen Polyurethanderivaten, assoziativen Cellulosederivaten, assoziativen Polyvinyllactamderivaten und assoziativen Derivaten ungesättigter Polysäuren, umfasst, für die Färbung von Keratinsubstanzen mit aufhellender Wirkung;
**dadurch gekennzeichnet, dass** der fluoreszierende Farbstoff aus den fluoreszierenden Farbstoffen, die zu den folgenden Familien gehören: Naphthalimiden; polykationischen fluoreszierenden Farbstoffen des Azomethin- oder Methintyps und 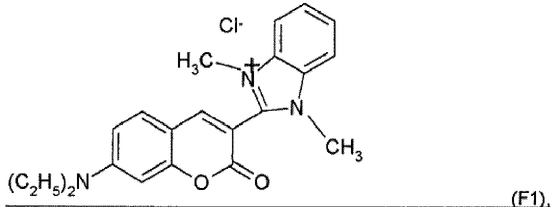 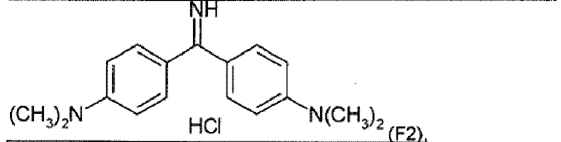 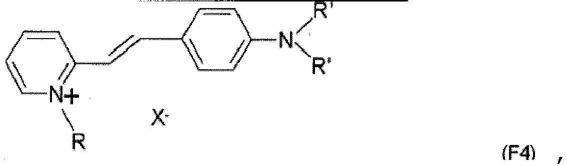 wobei in der Formel R für einen Methyl- oder Ethylrest steht; R' einen Methylrest, X⁻ ein Anion des Chlorid-, Iodid-, Sulfat-, Methosulfat-, Acetat-, Perchlorattyps darstellt, allein oder in Gemischen ausgewählt ist.

35. Verwendung nach dem vorhergehenden Anspruch, **dadurch gekennzeichnet, dass** der fluoreszierende Farbstoff ein Farbstoff im Orangebereich ist.

36. Verwendung nach einem der Ansprüche 34 oder 35, **dadurch gekennzeichnet, dass** der fluoreszierende Farbstoff zu einer maximalen Reflexion im Wellenlängenbereich von 500 bis 650 Nanometer und vorzugsweise im Wellenlängenbereich von 550 bis 620 Nanometer führt.

37. Verwendung nach einem der Ansprüche 34 bis 36, **dadurch gekennzeichnet, dass** der fluoreszierende Farbstoff aus der Gruppe ausgewählt ist, die von den Farbstoffen der folgenden Strukturen gebildet wird: 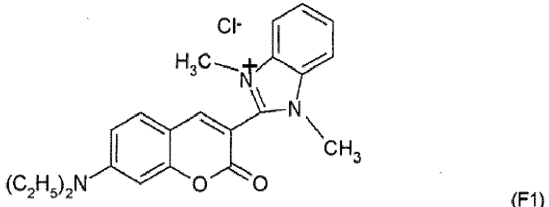 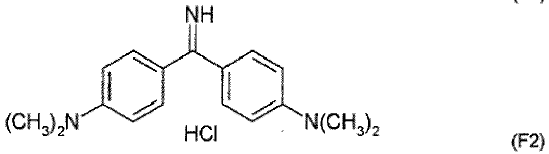 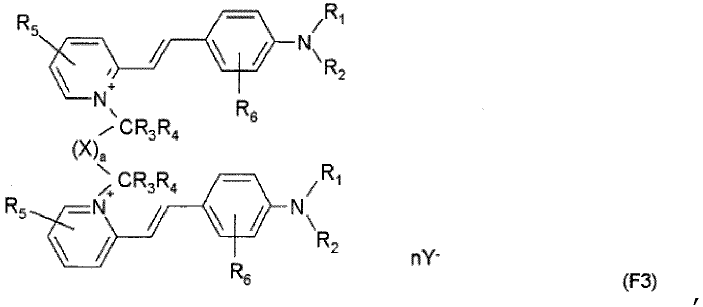 worin:
R₁, R₂, die gleich oder verschieden sind, für Folgendes stehen:
• ein Wasserstoffatom;
• einen geraden oder verzweigten Alkylrest mit 1 bis 10 Kohlenstoffatomen, vorzugsweise 1 bis 4 Kohlenstoffatomen, der gegebenenfalls durch mindestens ein Heteroatom und/oder eine Gruppe, die mindestens ein Heteroatom umfasst, unterbrochen und/oder substituiert ist und/oder mit mindestens einem Halogenatom substituiert ist;
• einen Aryl- oder Arylalkylrest, wobei der Arylrest 6 Kohlenstoffatome besitzt und der Alkylrest 1 bis 4 Kohlenstoffatome besitzt, wobei der Arylrest gegebenenfalls mit einem oder mehreren geraden oder verzweigten Alkylresten mit 1 bis 4 Kohlenstoffatomen substituiert ist, die gegebenenfalls durch mindestens ein Heteroatom und/oder eine Gruppe, die mindestens ein Heteroatom umfasst, unterbrochen und/oder substituiert sind, und/oder mit mindestens einem Halogenatom substituiert ist;
• wobei R₁ und R₂ gegebenenfalls derart verbunden sein können, dass sie mit dem Stickstoffatom einen Heterozyklus bilden, und ein oder mehrere weitere Heteroatome umfassen können, wobei der Heterozyklus gegebenenfalls mit mindestens einem geraden oder verzweigten Alkylrest substituiert ist, der vorzugsweise 1 bis 4 Kohlenstoffatome umfasst und gegebenenfalls durch mindestens ein Heteroatom und/oder eine Gruppe, die mindestens ein Heteroatom umfasst, unterbrochen und/oder substituiert ist und/oder mit mindestens einem Halogenatom substituiert ist;
• R₁ oder R₂ gegebenenfalls in einen Heterozyklus eingebunden sein können, der das Stickstoffatom und eines der Kohlenstoffatome der Phenylgruppe, die das Stickstoffatom trägt, umfasst;
R₃, R₄, die gleich sind oder nicht, ein Wasserstoffatom, einen Alkylrest mit 1 bis 4 Kohlenstoffatomen, darstellen;
R₅, die gleich sind oder nicht, ein Wasserstoffatom, ein Halogenatom, einen geraden oder verzweigten Alkylrest mit 1 bis 4 Kohlenstoffatomen, der gegebenenfalls durch mindestens ein Heteroatom unterbrochen ist, darstellen; R₆, die gleich sind oder nicht, ein Wasserstoffatom; ein Halogenatom; einen geraden oder verzweigten Alkylrest mit 1 bis 4 Kohlenstoffatomen, der gegebenenfalls durch mindestens ein Heteroatom und/oder eine Gruppe, die mindestens ein Heteroatom trägt, substituiert und/oder unterbrochen ist und/oder mit mindestens einem Halogenatom substituiert ist;
X Folgendes darstellt:
• einen geraden oder verzweigten Alkylrest mit 1 bis 14 Kohlenstoffatomen oder einen Alkenylrest mit 2 bis 14 Kohlenstoffatomen, der gegebenenfalls durch mindestens ein Heteroatom und/oder eine Gruppe, die mindestens ein Heteroatom umfasst, unterbrochen und/oder substituiert ist und/oder mit mindestens einem Halogenatom substituiert ist;
• einen 5 oder 6 Ringglieder umfassenden heterozyklischen Rest, der gegebenenfalls mit mindestens einem geraden oder verzweigten Alkylrest mit 1 bis 14 Kohlenstoffatomen, der gegebenenfalls mit mindestens einem Heteroatom substituiert ist; mit mindestens einem geraden oder verzweigten Aminoalkylrest mit 1 bis 4 Kohlenstoffatomen, der gegebenenfalls mit mindestens einem Heteroatom substituiert ist; mit mindestens einem Halogenatom substituiert ist;
• einen aromatischen oder einen kondensierten oder nicht-kondensierten diaromatischen Rest, der durch einen Alkylrest mit 1 bis 4 Kohlenstoffatomen getrennt ist oder nicht, wobei der oder die Arylrest(e) gegebenenfalls mit mindestens einem Halogenatom oder mit mindestens einem Alkylrest mit 1 bis 10 Kohlenstoffatomen, der gegebenenfalls durch mindestens ein Heteroatom und/oder eine Gruppe, die mindestens ein Heteroatom trägt, unterbrochen und/oder substituiert ist, substituiert ist;
• einen Dicarbonylrest;
• wobei die Gruppe X eine oder mehrere kationische Ladungen tragen kann;
a gleich 0 oder 1 ist;
Y⁻, die gleich sind oder nicht, ein organisches oder anorganisches Anion darstellen;
n eine ganze Zahl von mindestens gleich 2 und höchstens gleich der Anzahl an kationischen Ladungen, die in der fluoreszierenden Verbindung vorliegen, ist; 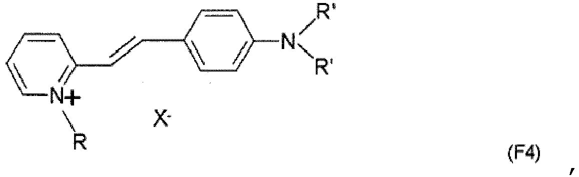
wobei in der Formel R für einen Methyl- oder Ethylrest steht; R' einen Methylrest, X⁻ ein Anion des Chlorid-, Iodid-, Sulfat-, Methosulfat-, Acetat-, Perchlorattyps darstellt.

38. Verwendung nach einem der Ansprüche 34 bis 37, **dadurch gekennzeichnet, dass** der oder die fluoreszierende(n) Farbstoff (e) in einer Konzentration, bezogen auf Gewicht, von 0,01 bis 20 Gew.-%, insbesondere von 0,05 bis 10 Gew.-%, vorzugsweise von 0,1 bis 5 Gew.-%, bezogen auf das Gesamtgewicht der Zusammensetzung, vorhanden sind.

39. Verwendung nach einem der Ansprüche 34 bis 38, **dadurch gekennzeichnet, dass** der Gehalt des oder der assoziativen Polymer(s/e) von 0,01 bis 10 Gew.-%, insbesondere von 0,1 bis 5 Gew.-%, bezogen auf das Gesamtgewicht der Zusammensetzung, variiert.

40. Verwendung nach einem der Ansprüche 34 bis 39, **dadurch gekennzeichnet, dass** es sich bei den Keratinsubstanzen um pigmentierte oder künstlich gefärbte Keratinfasern, insbesondere Haare, oder dunkle Haut handelt.

41. Verwendung nach Anspruch 40, **dadurch gekennzeichnet, dass** die Haare eine Farbtiefe gemäß der in dem Handbuch von Charles Zviak "Science des traitements capillaires", 1988, auf den Seiten 215 und 278 definierten Skala von weniger als oder gleich 6 und vorzugsweise weniger als oder gleich 4 aufweisen.

## Claims

1. Composition, **characterized in that** it comprises, in a cosmetically acceptable medium, at least one fluorescent dye soluble in the said medium and at least one associative polymer chosen from associative polyurethane derivatives, associative cellulose derivatives, associative polyvinyllactam derivatives and associative unsaturated polyacid derivatives; the composition not comprising, as fluorescent agent, 2-{2-[4-(dialkylamino)phenyl]ethenyl}-1-alkylpyridinium in which the alkyl radical of the pyridinium ring represents a methyl or ethyl radical and the alkyl radical of the benzene ring represents a methyl radical and in which the counterion is a halide, **characterized in that** the fluorescent dye is chosen from fluorescent dyes belonging to the following families: the naphthalimides; the polycationic fluorescent dyes of azomethine or methine type, and 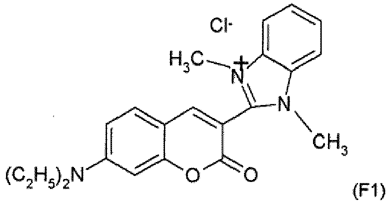 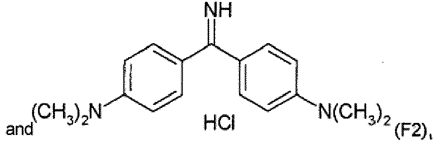 alone or as mixtures.

2. Composition according to Claim 1, **characterized in that** the associative polymer is of cationic, non-ionic, anionic or amphoteric nature.

3. Composition according to either of Claims 1 and 2, **characterized in that** the associative polyurethane is anionic and comprises at least one unit originating from a monomer of the α,β-monoethylenically unsaturated carboxylic acid type.

4. Composition according to either of Claims 1 and 2, **characterized in that** the associative polyurethane is cationic and corresponds to the following general formula (Ia) :
**R-X-(P)ₙ-[L-(Y)ₘ]ᵣ-L'-(P')ₚ-X'-R'** **(Ia)**
in which:
R and R', which are identical or different, represent a hydrophobic group or a hydrogen atom;
X and X', which are identical or different, represent a group comprising an amine functional group carrying or not carrying a hydrophobic group, or also the L" group;
L, L' and L", which are identical or different, represent a group derived from a diisocyanate;
P and P', which are identical or different, represent a group comprising an amine functional group carrying or not carrying a hydrophobic group; Y represents a hydrophilic group;
r is an integer of between 1 and 100, preferably between 1 and 50 and in particular between 1 and 25, n, m and p each have a value, independently of the others, between 0 and 1000;
the molecule containing at least one protonated or quaternized amine functional group and at least one hydrophobic group.

5. Composition according to either of Claims 1 and 2, **characterized in that** the associative polyurethane is a non-ionic polyurethane polyether.

6. Composition according to either of Claims 1 and 2, **characterized in that** the associative cellulose derivative is cationic and is chosen from celluloses or hydroxyethylcelluloses quaternized by a hydrophobic group.

7. Composition according to either of Claims 1 and 2, **characterized in that** the associative cellulose derivative is non-ionic and is chosen from hydroxyethylcelluloses modified by a hydrophobic group.

8. Composition according to either of Claims 1 and 2, **characterized in that** the associative polyvinyllactam derivative is cationic and comprises:
a) at least one monomer of vinyllactam or alkylvinyllactam type;
b) at least one monomer of following structures (II) or (III): 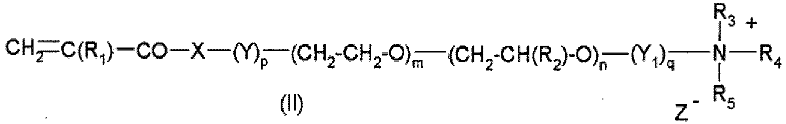 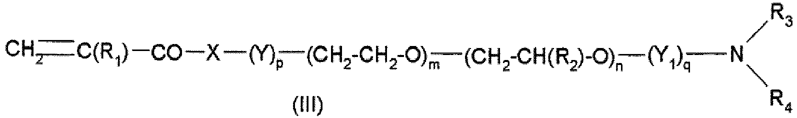 in which:
X denotes an oxygen atom or an NR₆ radical,
R₁ and R₆ denote, independently of one another, a hydrogen atom or a linear or branched C₁-C₅ alkyl radical,
R₂ denotes a linear or branched C₁-C₄ alkyl radical,
R₃, R₄ and R₅ denote, independently of one another, a hydrogen atom, a linear or branched C₁-C₃₀ alkyl radical or a radical of formula (IV):
-(Y₂)ᵣ-(CH₂-CH(R₇)-O)ₓ-R₈ (IV)
Y, Y₁ and Y₂ denote, independently of one another, a linear or branched C₂-C₁₆ alkylene radical,
R₇ denotes a hydrogen atom, a linear or branched C₁-C₄ alkyl radical or a linear or branched C₁-C₄ hydroxyalkyl radical,
Ra denotes a hydrogen atom or a linear or branched C₁-C₃₀ alkyl radical,
P, q and r denote, independently of one another, either the value zero or the value 1,
m and n denote, independently of one another, an integer ranging from 0 to 100,
x denotes an integer ranging from 1 to 100,
Z denotes an organic or inorganic acid anion,
with the proviso that:
- one at least of the R₃, R₄, R₅ or R₈ substituents denotes a linear or branched C₉-C₃₀ alkyl radical,
- if m or n is other than zero, then q is equal to 1,
- if m or n is equal to zero, then p or q is equal to 0.

9. Composition according to either of Claims 1 and 2, **characterized in that** the associative polyvinyllactam derivative is non-ionic and is chosen from copolymers of vinylpyrrolidone and of at least one hydrophobic monomer comprising a fatty chain.

10. Composition according to either of Claims 1 and 2, **characterized in that** the associative unsaturated polyacid derivative is an anionic polymer comprising at least one hydrophilic unit of olefinic unsaturated carboxylic acid type and at least one hydrophobic unit of unsaturated carboxylic acid C₁₀-C₃₀ alkyl ester type.

11. Composition according to either of Claims 1 and 2, **characterized in that** the associative unsaturated polyacid derivative is an anionic polymer comprising, among its monomers, an α,β-monethylenically unsaturated carboxylic acid and an ester of α,β-monoethylenically unsaturated carboxylic acid and of an oxyalkylenated fatty alcohol.

12. Composition according to either of Claims 10 and 11, **characterized in that** the carboxylic acid is acrylic acid or methacrylic acid.

13. Composition according to any one of Claims 1 to 12, **characterized in that** the content of the associative polymer or polymers varies from 0.01 to 10% by weight, more particularly from 0.1 to 5% by weight, of the total weight of the composition.

14. Composition according to any one of the preceding claims, **characterized in that** the fluorescent dye, which is optionally neutralized, is soluble in the cosmetic medium at at least 0.001 g/l, more particularly 0.5 g/l, preferably at least 1 g/l and, according to an even more preferred embodiment, at least 5 g/l at a temperature between 15 and 25°C.

15. Composition according to any one of the preceding claims, **characterized in that** the fluorescent dye is a dye within the range of the orangey colours.

16. Composition according to any one of the preceding claims, **characterized in that** the fluorescent dye results in a maximum of reflectance lying in the wavelength range extending from 500 to 650 nanometres and preferably in the wavelength range extending from 550 to 620 nanometres.

17. Composition according to any one of the preceding claims, **characterized in that** the fluorescent dye is chosen from the group formed by the dyes having the following structures: 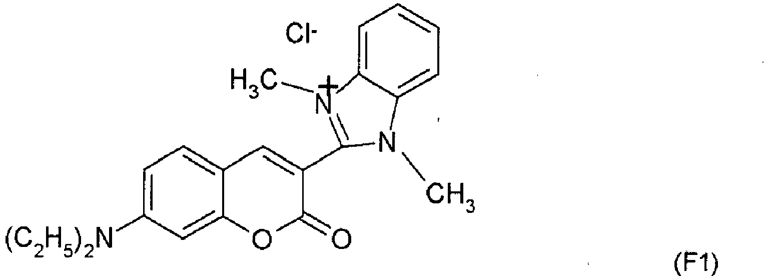 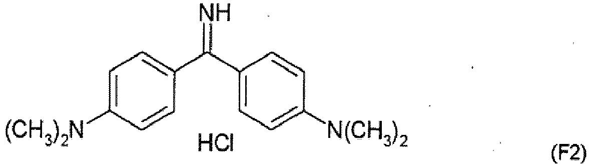 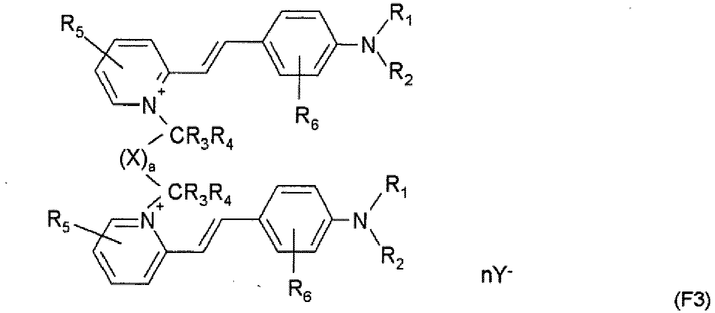 in which:
R₁ and R₂, which are identical or different, represent:
• a hydrogen atom;
• a linear or branched alkyl radical comprising from 1 to 10 carbon atoms, preferably from 1 to 4 carbon atoms, optionally interrupted and/or substituted by at least one heteroatom and/or group comprising at least one heteroatom and/or substituted by at least one halogen atom;
• an aryl or arylalkyl radical, the aryl group having 6 carbon atoms and the alkyl radical having from 1 to 4 carbon atoms; the aryl radical optionally being substituted by one or more linear or branched alkyl radicals comprising from 1 to 4 carbon atoms which are optionally interrupted and/or substituted by at least one heteroatom and/or group comprising at least one heteroatom and/or which is substituted by at least one halogen atom;
• R₁ and R₂ can optionally be connected so as to form a heterocycle with the nitrogen atom and comprise one or more other heteroatoms, the heterocycle optionally being substituted by at least one linear or branched alkyl radical preferably comprising from 1 to 4 carbon atoms and being optionally interrupted and/or substituted by at least one heteroatom and/or group comprising at least one heteroatom and/or substituted by at least one halogen atom;
• R₁ or R₂ can optionally be involved in a heterocycle comprising the nitrogen atom and one of the carbon atoms of the phenyl group carrying the said nitrogen atom;
R₃ and R₄, which are identical or different, represent a hydrogen atom or an alkyl radical comprising from 1 to 4 carbon atoms;
R₅, which are identical or different, represent a hydrogen atom, a halogen atom or a linear or branched alkyl radical comprising from 1 to 4 carbon atoms optionally interrupted by at least one heteroatom;
R₆, which are identical or different, represent a hydrogen atom, a halogen atom or a linear or branched alkyl radical comprising from 1 to 4 carbon atoms optionally substituted and/or interrupted by at least one heteroatom and/or group carrying at least one heteroatom and/or substituted by at least one halogen atom;
X represents:
• a linear or branched alkyl radical comprising from 1 to 14 carbon atoms or an alkenyl radical comprising from 2 to 14 carbon atoms optionally interrupted and/or substituted by at least one heteroatom and/or group comprising at least one heteroatom and/or substituted by at least one halogen atom;
• a 5- or 6-membered heterocyclic radical optionally substituted by at least one linear or branched alkyl radical comprising from 1 to 14 carbon atoms optionally substituted by at least one heteroatom; by at least one linear or branched aminoalkyl radical comprising from 1 to 4 carbon atoms optionally substituted by at least one heteroatom; or by at least one halogen atom;
• an aromatic radical or a diaromatic radical which is or is not fused and which is or is not separated by an alkyl radical comprising from 1 to 4 carbon atoms, the aryl radical or radicals optionally being substituted by at least one halogen atom or by at least one alkyl radical comprising from 1 to 10 carbon atoms optionally substituted and/or interrupted by at least one heteroatom and/or group carrying at least one heteroatom;
• a dicarbonyl radical;
• it being possible for the X group to carry one or more cationic charges;
a being equal to 0 or 1;
Y⁻, which are identical or different, representing an organic or inorganic anion;
n being an integer at least equal to 2 and at most equal to the number of cationic charges present in the fluorescent compound.

18. Composition according to any one of the preceding claims, **characterized in that** the fluorescent dye or dyes are present in a concentration by weight of between 0.01 and 20% by weight, more particularly of 0.05 and 10% by weight, preferably between 0.1 and 5% by weight, with respect to the total weight of the composition.

19. Composition according to any one of the preceding claims, **characterized in that** it additionally comprises at least one nonfluorescent additional direct dye of non-ionic, cationic or anionic nature.

20. Composition according to Claim 19, **characterized in that** the additional direct dyes are chosen from nitrobenzene dyes, azo, anthraquinone, naphthoquinone, benzoquinone, phenothiazine, indigoid, xanthene, phenanthridine or phthalocyanine dyes and also dyes derived from triarylmethane, or their mixtures.

21. Composition according to either of Claims 19 and 20, **characterized in that** the additional direct dye or dyes represent from 0.0005 to 12% by weight, preferably from 0.005 to 6% by weight, of the total weight of the composition.

22. Composition according to any one of the preceding claims, **characterized in that** it is provided in the form of a lightening and dyeing shampoo.

23. Composition according to any one of the preceding claims, **characterized in that** it comprises at least one oxidation base chosen from para-phenylenediamines, bisphenylalkylenediamines, para-aminophenols, ortho-aminophenols and heterocyclic bases or their addition salts with an acid or with an alkaline agent.

24. Composition according to Claim 23, **characterized in that** the oxidation base or bases represent from 0.0005 to 12% by weight, more particularly from 0.005 to 6% by weight, of the total weight of the composition.

25. Composition according to either one of Claims 23 and 24, **characterized in that** it comprises at least one coupler chosen from meta-phenylenediamines, meta-aminophenols, meta-diphenols and heterocyclic couplers or their addition salts with an acid or with an alkaline agent.

26. Composition according to Claim 25, **characterized in that** the coupler or couplers represent from 0.0001 to 10% by weight, more particularly from 0.005 to 5% by weight, of the total weight of the composition.

27. Ready-for-use composition, **characterized in that** it comprises the composition according to one of Claims 1 to 26 and at least one oxidizing agent.

28. Composition according to the preceding claim, **characterized in that** the oxidizing agent is chosen from hydrogen peroxide, urea hydrogen peroxide, alkali metal bromates, persalts, such as perborates and persulfates, and enzymes, such as peroxidases and two- or four-electron oxidoreductases.

29. Method for colouring, with a lightening effect, human keratinous fibres, **characterized in that** the following stages are carried out:
a) a composition as defined according to any one of Claims 1 to 28 is applied to the said fibres for a time sufficient to develop the desired colouring and desired lightening,
b) the fibres are optionally rinsed,
c) the fibres are optionally washed with a shampoo and rinsed,
d) the fibres are dried or allowed to dry.

30. Method according to Claim 29, **characterized in that** it comprises a preliminary stage consisting in storing, in separate form, on the one hand, a composition according to one of Claims 1 to 21 and 23 to 26 and, on the other hand, a composition including, in a cosmetically acceptable medium, at least one oxidizing agent, and in then mixing them at the time of use, before applying this mixture to the fibres for a time sufficient to develop the desired colouring, after which they are rinsed, optionally washed with a shampoo, rinsed again and dried.

31. Method according to either one of Claims 29 and 30, **characterized in that** the composition is applied to hair exhibiting a tone depth, according to the scale defined in the work by Charles Zviak, "Science des traitements capillaires [Science of Hair Treatments]", 1988, on pages 215 and 278, of less than or equal to 6 and preferably of less than or equal to 4.

32. Method according to one of Claims 29 to 31, **characterized in that** the human keratinous fibres are artificially pigmented or coloured.

33. Method for colouring, with a lightening effect, dark skin, **characterized in that** the composition according to any one of Claims 1 to 21 is applied to the skin and then the skin is dried or allowed to dry.

34. Use of a composition comprising, in a cosmetically acceptable medium, at least one fluorescent dye which is soluble in the said medium and at least one associative polymer chosen from associative polyurethane derivatives, associative cellulose derivatives, associative polyvinyllactam derivatives and associative unsaturated polyacid derivatives in the colouring with lightening effect of keratinous substances;
**characterized in that** the fluorescent dye is chosen from fluorescent dyes belonging to the following families: the naphthalimides; the polycationic fluorescent dyes of azomethine or methine type, 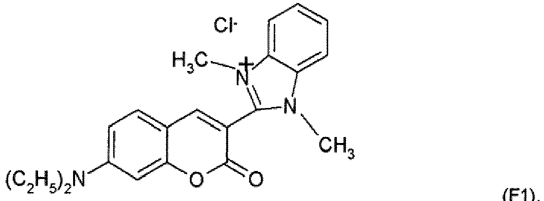 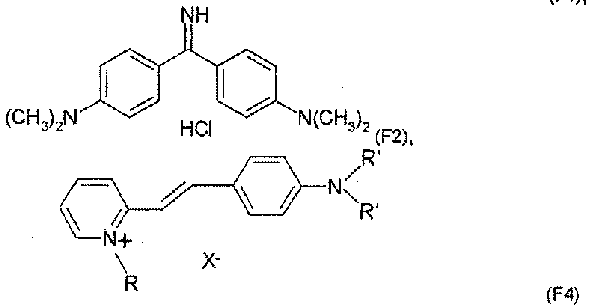 in which formula R represents a methyl or ethyl radical, R' represents a methyl radical and X⁻ represents an anion of the chloride, iodide, sulfate, methosulfate, acetate or perchlorate type, alone or as mixtures.

35. Use according to the preceding claim, **characterized in that** the fluorescent dye is a dye within the range of the orangey colours.

36. Use according to either one of Claims 34 and 35, **characterized in that** the fluorescent dye results in a maximum of reflectance lying within the wavelength range extending from 500 to 650 nanometres and preferably within the wavelength range extending from 550 to 620 nanometres.

37. Use according to any one of Claims 34 to 36, **characterized in that** the fluorescent dye is chosen from the group formed by the dyes having the following structures: 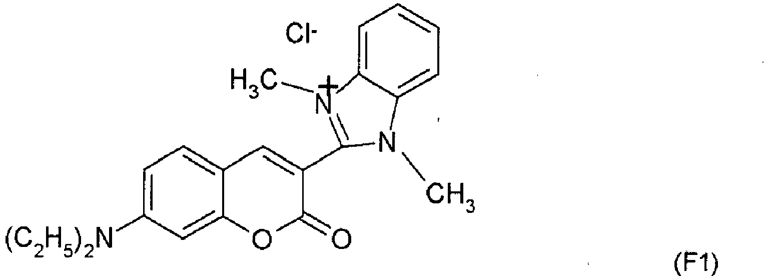 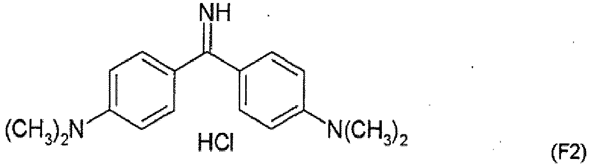 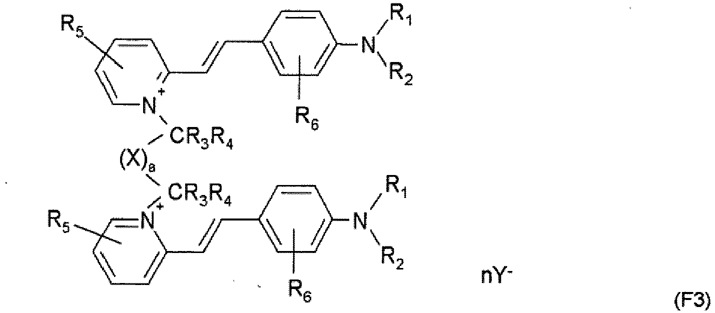 in which:
R₁ and R₂, which are identical or different, represent:
• a hydrogen atom;
• a linear or branched alkyl radical comprising from 1 to 10 carbon atoms, preferably from 1 to 4 carbon atoms, optionally interrupted and/or substituted by at least one heteroatom and/or group comprising at least one heteroatom and/or substituted by at least one halogen atom;
• an aryl or arylalkyl radical, the aryl group having 6 carbon atoms and the alkyl radical having from 1 to 4 carbon atoms; the aryl radical optionally being substituted by one or more linear or branched alkyl radicals comprising from 1 to 4 carbon atoms which are optionally interrupted and/or substituted by at least one heteroatom and/or group comprising at least one heteroatom and/or which is substituted by at least one halogen atom;
• R₁ and R₂ can optionally be connected so as to form a heterocycle with the nitrogen atom and comprise one or more other heteroatoms, the heterocycle optionally being substituted by at least one linear or branched alkyl radical preferably comprising from 1 to 4 carbon atoms and being optionally interrupted and/or substituted by at least one heteroatom and/or group comprising at least one heteroatom and/or substituted by at least one halogen atom;
• R₁ or R₂ can optionally be involved in a heterocycle comprising the nitrogen atom and one of the carbon atoms of the phenyl group carrying the said nitrogen atom;
R₃ and R₄, which are identical or different, represent a hydrogen atom or an alkyl radical comprising from 1 to 4 carbon atoms;
R₅, which are identical or different, represent a hydrogen atom, a halogen atom or a linear or branched alkyl radical comprising from 1 to 4 carbon atoms optionally interrupted by at least one heteroatom;
R₆, which are identical or different, represent a hydrogen atom, a halogen atom or a linear or branched alkyl radical comprising from 1 to 4 carbon atoms optionally substituted and/or interrupted by at least one heteroatom and/or group carrying at least one heteroatom and/or substituted by at least one halogen atom;
X represents:
• a linear or branched alkyl radical comprising from 1 to 14 carbon atoms or an alkenyl radical comprising from 2 to 14 carbon atoms optionally interrupted and/or substituted by at least one heteroatom and/or group comprising at least one heteroatom and/or substituted by at least one halogen atom;
• a 5- or 6-membered heterocyclic radical optionally substituted by at least one linear or branched alkyl radical comprising from 1 to 14 carbon atoms optionally substituted by at least one heteroatom; by at least one linear or branched aminoalkyl radical comprising from 1 to 4 carbon atoms optionally substituted by at least one heteroatom; or by at least one halogen atom;
• an aromatic radical or a diaromatic radical which is or is not fused and which is or is not separated by an alkyl radical comprising from 1 to 4 carbon atoms, the aryl radical or radicals optionally being substituted by at least one halogen atom or by at least one alkyl radical comprising from 1 to 10 carbon atoms optionally substituted and/or interrupted by at least one heteroatom and/or group carrying at least one heteroatom;
• a dicarbonyl radical;
• it being possible for the X group to carry one or more cationic charges;
a being equal to 0 or 1;
Y⁻, which are identical or different, representing an organic or inorganic anion;
n being an integer at least equal to 2 and at most equal to the number of cationic charges present in the fluorescent compound, 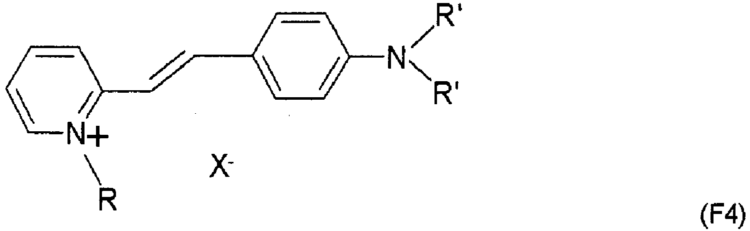 in which formula R represents a methyl or ethyl radical, R' represents a methyl radical and X⁻ represents an anion of the chloride, iodide, sulfate, methosulfate, acetate or perchlorate type.

38. Use according to any one of Claims 34 to 37, **characterized in that** the fluorescent dye or dyes are present in a concentration by weight ranging from 0.01 to 20% by weight, more particularly from 0.05 to 10% by weight and preferably from 0.1 to 5% by weight, with respect to the total weight of the composition.

39. Use according to one of Claims 34 to 38, **characterized in that** the content of the associative polymer or polymers varies from 0.01 to 10% by weight, more particularly from 0.1 to 5% by weight, of the total weight of the composition.

40. Use according to one of Claims 34 to 39, **characterized in that** the keratinous substances are artificially pigmented or coloured keratinous fibres, in particular hair, or dark skin.

41. Use according to Claim 40, **characterized in that** the hair exhibits a tone depth, according to the scale defined in the work by Charles Zviak, "Science des traitements capillaires [Science of Hair Treatments]", 1988, on pages 215 and 278, of less than or equal to 6 and preferably of less than or equal to 4.
